Europäisches Patentamt

European Patent Office

Office européen des brevets

(19)

(11) EP 1 153 932 A1

(12) EUROPEAN PATENT APPLICATION
published in accordance with Art. 158(3) EPC

(43) Date of publication:
14.11.2001 Bulletin 2001/46

(51) Int Cl.⁷: **C07K 14/705**, C12N 15/12,
C12N 5/10, C12P 21/02,
C07K 16/28, C12P 21/08,
A61K 39/395, G01N 33/50,
C12Q 1/00, C07K 14/46,
C12Q 1/68

(21) Application number: 00904030.4

(22) Date of filing: 18.02.2000

(86) International application number:
PCT/JP00/00927

(87) International publication number:
WO 00/49046 (24.08.2000 Gazette 2000/34)

(84) Designated Contracting States:
AT BE CH CY DE DK ES FI FR GB GR IE IT LI LU
MC NL PT SE
Designated Extension States:
AL LT LV MK RO SI

(30) Priority: 19.02.1999 JP 4133699
06.05.1999 JP 12576899

(71) Applicant: Takeda Chemical Industries, Ltd.
Osaka-shi, Osaka 541-0045 (JP)

(72) Inventors:
• WATANABE, Takuya
  Osaka-shi, Osaka 532-0033 (JP)
• TERAO, Yasuko
  Tsukuba-shi, Ibaraki 305-0034 (JP)
• SHINTANI, Yasushi
  Tsukuba-shi, Ibaraki 305-0821 (JP)

(74) Representative: Lederer, Franz, Dr. et al
Lederer, Keller & Riederer
Patentanwälte
Prinzregentenstrasse 16
80538 München (DE)

(54) **NOVEL G PROTEIN-COUPLED RECEPTOR PROTEIN AND DNA THEREOF**

(57) The present invention relates to a human-derived G protein-coupled receptor protein, its partial peptides, or salts thereof, a nucleic acid encoding the receptor protein and its derivatives, a nucleic acid having an antisense sequence to the base sequence encoding the receptor protein and its derivatives, methods of manufacturing the G protein-coupled receptor protein, methods for determining a ligand to the G protein-coupled receptor protein, screening methods/screening kits for a compound that alters the binding property between a ligand and the G protein-coupled receptor protein, a compound obtainable by the screening or its salts, an antibody to the G protein-coupled receptor protein, etc.

The human (human hippocampus)-derived G protein-coupled receptor protein of this invention or the nucleic acid and its derivatives encoding the protein can be used for ① determination of ligands (agonists) to the receptor protein of the present invention; ② prophylactic and/or therapeutic agents for diseases associated with dysfunction of the G protein-coupled receptor protein of the present invention; ③ gene diagnostic agents; ④ quantification of a ligand to the G protein-coupled receptor protein of the present invention; ⑤ screening of compounds (agonists, antagonists, etc.) that alter the binding property between the G protein-coupled receptor protein of the present invention and ligands; ⑥ prophylactic and/or therapeutic agents for various diseases, comprising the compounds (agonists, antagonists, etc.) that alter the binding property between the G protein-coupled receptor protein of the present invention and ligands; ⑦ quantification of the receptor protein of the present invention, its partial peptides, or salts thereof; ⑧ neutralization by antibodies to the receptor protein of the present invention, its partial peptides, or salts thereof; and the like.

EP 1 153 932 A1

## Description

FIELD OF THE INVENTION

The present invention relates to a novel protein derived from human (human hippocampus) or its salts and DNA encoding the same, etc.

BACKGROUND ART

Physiological active substances such as various hormones and neurotransmitters regulate the biological function via specific receptor proteins present on cell membranes. Many of these receptor proteins are coupled with guanine nucleotide-binding protein (hereinafter sometimes simply referred to as G protein) and mediate the intracellular signal transduction via activation of G protein. These receptor proteins possess the common structure containing seven transmembrane domains and are thus collectively referred to as G protein-coupled receptors or seven-transmembrane receptors (7TMR).

G protein-coupled receptor proteins present on the cell surface of each functional cell and organ in the body, and play important physiological roles as the target of the molecules that regulate the functions of the cells and organs, e.g., hormones, neurotransmitters, physiologically active substances and the like. Receptors transmit signals to cells via binding with physiologically active substances, and the signals induce various reactions such as activation and inhibition of the cells.

To clarify the relationship between substances that regulate complex biological functions in various cells and organs, and their specific receptor proteins, in particular, G protein-coupled receptor proteins, would elucidate the functional mechanisms in various cells and organs in the body to provide a very important means for development of drugs closely associated with the functions.

For example, in various organs, their physiological functions are controlled in vivo through regulation by many hormones, hormone-like substances, neurotransmitters or physiologically active substances. In particular, physiologically active substances are found in numerous sites of the body and regulate the physiological functions through their corresponding receptor proteins. However, it is supposed that many unknown hormones, neurotransmitters or many other physiologically active substances still exist in the body and, as to their receptor proteins, many of these proteins have not yet been reported. In addition, it is still unknown if there are subtypes of known receptor proteins.

It is very important for development of drugs to clarify the relationship between substances that regulate elaborated functions in vivo and their specific receptor proteins. Furthermore, for efficient screening of agonists and antagonists to receptor proteins in development of drugs, it is required to clarify functional mechanisms of receptor protein genes expressed in vivo and express the genes in an appropriate expression system.

In recent years, random analysis of cDNA sequences has been actively studied as a means for analyzing genes expressed in vivo. The sequences of CDNA fragments thus obtained have been registered on and published to databases as Expressed Sequence Tag (EST). However, since many ESTs contain sequence information only, it is difficult to predict their functions from the information.

Substances that inhibit binding between G protein-coupled proteins and physiologically active substances (i.e., ligands) and substances that bind and induce signals similar to those induced by physiologically active substances (i.e., ligands) have been used as pharmaceuticals, as antagonists and agonists specific to the receptors, that regulate the biological functions. Therefore, discovery and gene cloning (e.g., cDNA) of a novel G protein-coupled receptor that can be targeted for pharmaceutical development are very important means in search for a specific ligand, agonist, and antagonist of the novel G protein-coupled receptor.

However, not all G protein-coupled receptors have been discovered. There are unknown G protein-coupled receptors and many of these receptors in which the corresponding ligands are yet unidentified are called orphan receptors. Therefore, search and functional elucidation of a novel G protein-coupled receptor is awaited.

G protein-coupled receptors are useful in searching for a novel physiological active substance (i.e., ligand) using the signal transduction activity as the index and in search for agonists and antagonists of the receptor. Even if no physiological ligand is found, agonists and antagonist of the receptor may be prepared by analyzing the physiological action of the receptor through inactivation experiment of the receptor (knockout animal). Ligands, agonists, antagonists, etc. of the receptor are expected to be used as prophylactic/therapeutic and diagnostic agents for diseases associated with dysfunction of the G protein-coupled receptor.

Lowering or accentuation in functions of the G protein coupled receptor due to genetic aberration of the receptor in vivo causes some disorders in many cases. In this case, the G protein coupled receptor may be used not only for administration of antagonists or agonists of the receptor, but also for gene therapy by transfer of the receptor gene into the body (or some specific organs) or by introduction of the antisense nucleic acid of the receptor gene into the body (or the specific organ). In the gene therapy, information on the base sequence of the receptor gene is essentially required for investigating deletion or mutation in the gene. The receptor gene is also applicable as prophylactic/therapeutic and diagnostic agents for diseases associated with dysfunction of the receptor.

The present invention provides a novel and useful G protein-coupled receptor protein as described above. That is, the present invention provides a novel G protein-coupled receptor protein, its partial peptides and salts thereof, as well as polynucleotides (DNA and RNA, and derivatives thereof) containing the polynucleotides (DNA and RNA, and derivatives thereof) encoding the G protein-coupled receptor protein or its partial peptides, recombinant vectors containing the polynucleotides, transformants bearing the recombinant vectors, methods for manufacturing the G protein-coupled receptor protein or its salts, antibodies to the G protein-coupled receptor protein, its partial peptides and salts thereof, compounds that alter the expression level of said G protein-coupled receptor protein, methods for determination of ligands to the G protein-coupled receptor protein, methods for screening the compounds (antagonists and agonists) or salts thereof that alter the binding property of ligands and the G protein-coupled receptor protein, kits for use in the screening methods, compounds (antagonists and agonists) or salts thereof that alter the binding property of ligands obtainable by the screening methods or obtainable using the screening kits and the G protein-coupled receptor protein, and pharmaceutical compositions comprising the compounds (antagonists and agonists) that alter the binding property of ligands to the G protein-coupled receptor protein, or compounds or salts thereof that alter the expression level of the G protein-coupled receptor protein.

DISCLOSURE OF THE INVENTION

As a result of extensive investigations, the present inventors have succeeded in isolating cDNAs encoding novel G protein-coupled receptor proteins derived from human (human hippocampus), based on the EST information prepared by the degenerated PCR technique, and in sequencing the full-length base sequences. When the base sequences were translated into the amino acid sequences, 1 to 7 transmembrane domains were found to be on the hydrophobic plot, establishing that the proteins encoded by these cDNAs are seven-transmembrane type G protein-coupled receptor proteins.

Based on these findings, the present inventors have continued further extensive studies and as a result, have come to accomplish the present invention.

Thus, the present invention relates to the following features.

(1) A protein containing the same or substantially the same amino acid sequence as the amino acid sequence represented by SEQ ID NO:1, or a salt thereof.
(2) A partial peptide of the protein according to (1), or a salt thereof.
(3) A polynucleotide containing a polynucleotide having a base sequence encoding the protein according to (1).
(4) A polynucleotide according to (3), which is DNA.

(5) A polynucleotide according to (3), which is represented by SEQ ID NO:2.
(6) A recombinant vector containing the polynucleotide according to (3).
(7) A transformant transformed with the recombinant vector according to (6).
(8) A method of manufacturing the protein or its salt according to (1), which comprises culturing the transformant according to (7) and accumulating the protein according to (1).
(9) An antibody to the protein according to (1), the partial peptide according to (3), or a salt of said protein or partial peptide.
(10) An antibody according to (9), which is a neutralizing antibody capable of inactivating signal transduction of the protein according to (1).
(11) A diagnostic composition comprising an antibody according to (9).
(12) A ligand to the protein or its salt according to (1), which is obtainable using the protein according to (1) or the partial peptide according to (2), or a salt of said protein or partial peptide.
(13) A pharmaceutical composition comprising the ligand according to (12).
(14) A method of determining a ligand to the protein or its salt according to (1), which comprises using the protein according to (1) or the partial peptide according to (2), or a salt of said protein or partial peptide.
(15) A method of screening a compound that alters the binding property between a ligand and the protein or its salt according to (1), wherein the protein according to (1), the partial peptide according to (2), or a salt of said protein or partial peptide.
(16) A kit for screening a compound or its salt that alters the binding property between a ligand and the protein or its salt according to (1), comprising the protein according to (1) or the partial peptide according to (2), or a salt of said protein or partial peptide.
(17) A compound or its salt that alters the binding property between a ligand and the protein or its salt according to (1), which is obtainable using the screening method according to (15) or the screening kit according to (16).
(18) A pharmaceutical composition comprising a compound or its salt that alters the binding property between a ligand and the protein or its salt according to (1), which is obtainable using the screening method according to (15) or the screening kit according to (16).
(19) A polynucleotide that hybridizes to the polynucleotide according to (3) under a highly stringent condition.
(20) A polynucleotide comprising a base sequence complementary to the polynucleotide according to (3) or a part of the base sequence.
(21) A method of quantifying mRNA of the protein according to (1), which comprises using the polynucleotide according to (3) or a part of the polynucleotide.

(22) A method of quantifying the protein according to (1), which comprises using the antibody according to (9).

(23) A diagnostic method for a disease associated with functions of the protein according to (1), which comprises using the quantification method according to (21) or (22).

(24) A method of screening a compound or its salt that alters the expression level of the protein according to (1), which comprises using the quantification method according to (21).

(25) A method of screening a compound or its salt that alters the amount of the protein according to (1) in cell membrane, which comprises using the quantification method according to (22).

(26) A compound or its salt that alters the expression level of the protein according to (1), which is obtainable using the screening method according to (24).

(27) A compound or its salt that alters the amount of the protein according to (1) in cell membrane, which is obtainable using the screening method according to (25).

The present invention further relates to the following features.

(28) A protein or its salt according to (1), wherein said protein contains ① the amino acid sequence shown by SEQ ID NO:1, of which at least 1 or 2 (preferably approximately 1 to 30, more preferably approximately 1 to 9, most preferably several (1 to 5)) amino acids are deleted, ② the amino acid sequence shown by SEQ ID NO:1, to which at least 1 or 2 (preferably approximately 1 to 30, more preferably approximately 1 to 10, most preferably several (1 to 5)) amino acids are added; ③ the amino acid sequence shown by SEQ ID NO:1, in which at least 1 or 2 (preferably approximately 1 to 30, more preferably approximately 1 to 10, most preferably several (1 to 5)) amino acids are substituted; or ④ the amino acid sequence containing a combination of these amino acid sequences.

(29) A method of determining a ligand according to (14), which comprises contacting the protein or its salt according to (1) or the partial peptide or its salt according to (2) with a test compound.

(30) A method of determining a ligand according to (29), in which said ligand is, for example, angiotensin, bombesin, canavinoid, cholecystokinin, glutamine, serotonin, melatonin, neuropeptide Y, an opioid, a purine, vasopressin, oxytocin, PACAP, secretin, glucagon, calcitnonin, adrenomedulin, somatostatin, GHRH, CRF, ACTH, GRP, PTH, vasoactive intestinal and related polypeptide (VIP), somatostatin, dopamine, motilin, amylin, bradykinin, calcitonin gene-related peptide (CGRP), a leukotriene, pancreastatin, a prostaglandin, thromboxane, adenosine, adrenaline, an $\alpha$- and $\beta$-chemokine (e.g., IL-8, GRO$\alpha$, GRO$\beta$, GRO$\gamma$, NAP-2, ENA-78, PF4, IP10, GCP-2, MCP-1, HC14, MCP-3, I-309, MIP1-$\alpha$, MIP-1$\beta$, RANTES, etc.), endothelin, enterogastrin, histamine, neurotensin, TRH, pancreatic polypeptide, or galanin.

(31) A method of screening according to (15), in which (i) contact of a ligand with the protein or its salt according to (1) or the partial peptide or its salt according to (2) is compared with (ii) contact of the ligand and a test compound with the protein or its salt according to (1) or the partial peptide or its salt according to (2).

(32) A method of screening a compound or its salt that alters the binding property between a ligand and the protein or its salt according to (1), which comprises measuring the amounts of a labeled ligand bound to the protein or its salt according to (1) or to the partial peptide or its salt according to (2), (i) when the labeled ligand is brought in contact with the protein or its salt according to (1) or with the partial peptide or its salt according to (2), and (ii) when the labeled ligand and a test compound are brought in contact with the protein or its salt according to (1) or with the partial peptide or its salt according to (2); and comparing the amounts measured in (i) and (ii).

(33) A method of screening a compound or its salt that alters the binding property between a ligand and the protein or its salt according to (1), which comprises measuring the amounts of a labeled ligand bound to a cell containing the protein according to (1), (i) when the labeled ligand is brought in contact with the cell containing the protein according to (1), and (ii) when the labeled ligand and a test compound are brought in contact with the cell containing the protein according to (1); and comparing the amounts measured in (i) and (ii).

(34) A method of screening a compound or its salt that alters the binding property between a ligand and the protein or its salt according to (1), which comprises measuring the amounts of a labeled ligand bound to a cell membrane fraction containing the protein according to (1), (i) when the labeled ligand is brought in contact with the cell membrane fraction, and (ii) when the labeled ligand and a test compound are brought in contact with the cell membrane fraction; and comparing the amounts measured in (i) and (ii).

(35) A method of screening a compound or its salt that alters the binding property between a ligand and the protein or its salt according to (1), which comprises measuring the amounts of a labeled ligand bound to a protein expressed in a cell membrane, (i) when the labeled ligand is brought in contact with the protein expressed in a cell membrane of the transformant according to (7) by culturing the transformant and (ii) when the labeled ligand and a test compound are brought in contact with the protein expressed in a cell membrane of the transformant according to (7) by culturing the transformant; and comparing the amounts measured in (i) and (ii).

(36) A method of screening a compound or its salt that alters the binding property between a ligand and the protein or its salt according to (1), which comprises measuring the protein-mediated cell stimulating activities, (i) when a compound that activates the protein or its salt according to (1) is brought in contact with a cell containing the protein according to (1), and (ii) when a compound that activates the protein or its salt according to (1) and a test compound are brought in contact with a cell containing the protein according to (1); and comparing the activities measured in (i) and (ii).

(37) A method of screening a compound or its salt that alters the binding property between a ligand and the protein or its salt according to (1), which comprises measuring the protein-mediated cell stimulating activities, when a compound that activates the protein or its salt according to (1) is brought in contact with a protein expressed in a cell membrane of the transformant according to (7) by culturing the transformant, and when the compound that activates the protein or its salt according to (1) and a test compound are brought in contact with the protein expressed in a cell membrane of the transformant according to (7) by culturing the transformant; and comparing the protein-mediated activities measured in (i) and (ii).

(38) A method of screening according to (36) or (37), in which said compound that activates the protein according to (1) is angiotensin, bombesin, canavinoid, cholecystokinin, glutamine, serotonin, melatonin, neuropeptide Y, an opioid, a purine, vasopressin, oxytocin, PACAP, secretin, glucagon, calcitnonin, adrenomedulin, somatostatin, GHRH, CRF, ACTH, GRP, PTH, vasoactive intestinal and related polypeptide (VIP), somatostatin, dopamine, motilin, amylin, bradykinin, calcitonin gene-related peptide (CGRP), a leukotriene, pancreastatin, a prostaglandin, thromboxane, adenosine, adrenaline, an $\alpha$- and $\beta$-chemokine (e.g., IL-8, GRO$\alpha$, CRO$\beta$, GRO$\gamma$, NAP-2, ENA-78, PF4, IP10, GCP-2, MCP-1, HC14, MCP-3, I-309, MIP1-$\alpha$, MIP-1$\beta$, RANTES, etc.), endothelin, enterogastrin, histamine, neurotensin, TRH, pancreatic polypeptide, or galanin.

(39) A compound or its salt that alters the binding property between a ligand and the protein or its salt according to (1), which is obtainable by the screening methods according to (31) through (38).

(40) A pharmaceutical composition comprising a compound or its salt that alters the binding property between a ligand and the protein or its salt according to (1), which is obtainable by the screening methods according to (31) through (38).

(41) A kit for screening according to (16), comprising a cell containing the protein according to (1).

(42) A screening kit according to (16), comprising a cell membrane fraction containing the protein according to (1).

(43) A screening kit according to (16), comprising a protein expressed on the cell membrane of the transformant according to (7) by culturing the transformant.

(44) A compound or its salt that alters the binding property of a ligand and the protein or its salt according to (1), which is obtainable using the screening kits according to (41) through (43).

(45) A pharmaceutical composition comprising a compound or its salt that alters the binding property of a ligand compound or its salt that alters the binding property between a ligand and the protein or its salt according to (1), which is obtainable using the screening kits according to (41) through (43).

(46) A method of quantifying the protein according to (1), the partial peptide according to (2), or a salt thereof, which comprises contacting the antibody according to (9) with the protein according to (1), the partial peptide according to (2), or a salt thereof.

(47) A method of quantifying the protein according to (1), the partial peptide according to (2) or salts thereof in a test fluid, which comprises competitively reacting the antibody according to (9) with a test fluid and a labeled form of the protein according to (1), the partial peptide according to (2) or salts thereof; and measuring the ratios bound to the antibody of the labeled form of the protein according to (1), the partial peptide or its salts according to (2).

(48) A method of quantifying the protein according to (1), the partial peptide according to (2), or salts thereof in a test fluid, which comprises reacting a test fluid simultaneously or sequentially with the antibody according to (9) immobilized on a carrier and the labeled antibody according to (9), and then measuring the activity of the label on the immobilizing carrier.

BRIEF DESCRIPTION OF THE DRAWINGS

FIG. 1 shows the base sequence of the DNA encoding novel human hippocampus-derived G protein-coupled receptor protein hSLT of the present invention obtained in Example 1, and the amino acid sequence deduced from the base sequence (continued on FIG. 2).

FIG. 2 shows the base sequence of the DNA encoding novel human hippocampus-derived G protein-coupled receptor protein hSLT of the present invention obtained in Example 1, and the amino acid sequence deduced from the base sequence (continued from FIG. 1).

FIG. 3 shows the hydrophobic plotting of novel human hippocampus-derived G protein-coupled receptor protein hSLT of the present invention prepared based on the amino acid sequence shown in FIGS. 1 and 2.

## BEST MODE FOR CARRYING OUT THE INVENTION

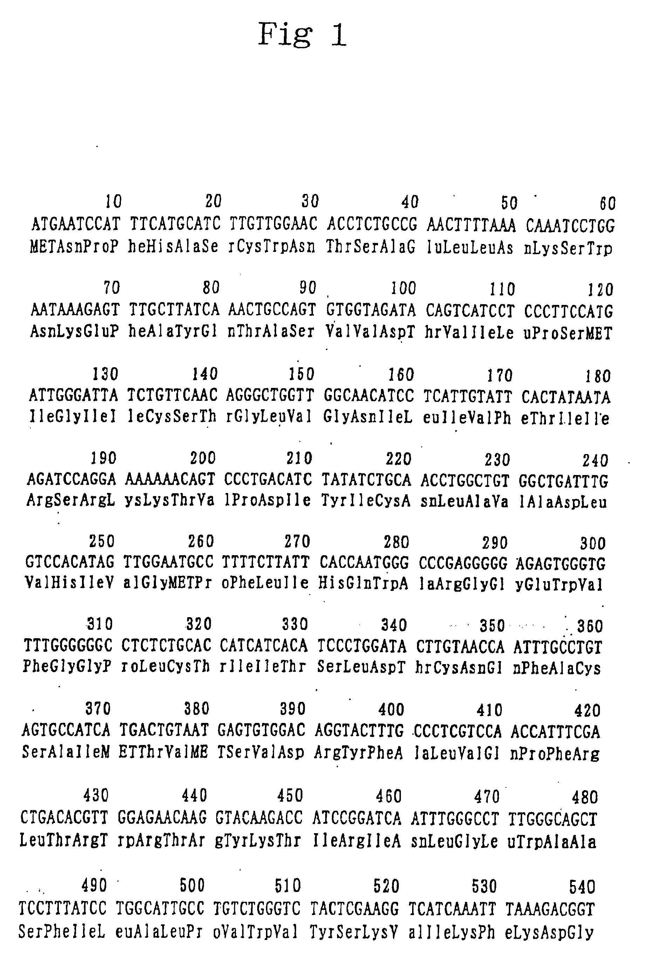

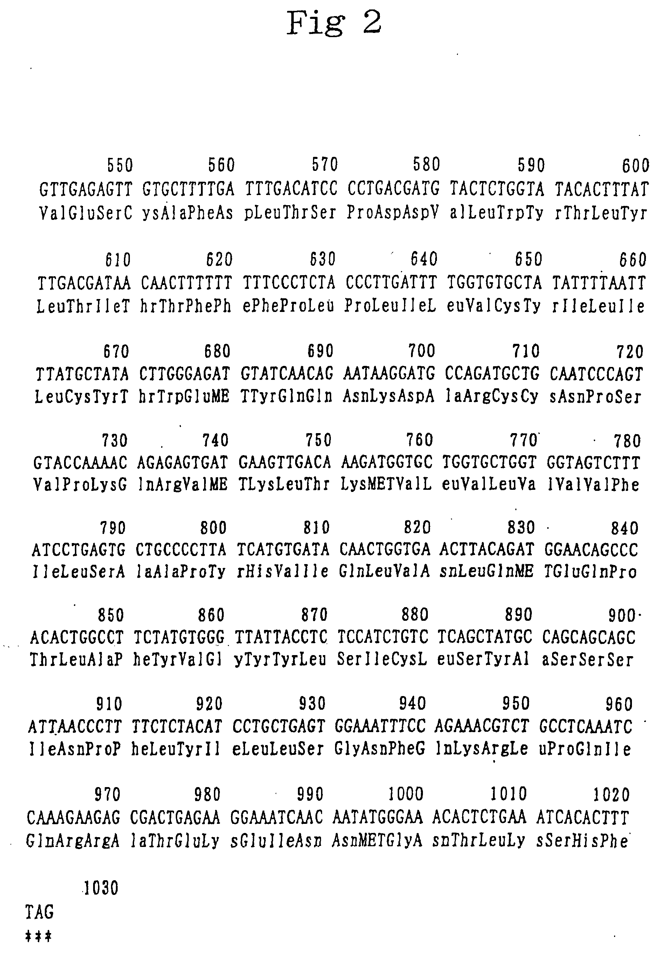

The G protein-coupled receptor protein of the present invention (hereinafter sometimes merely referred to as the receptor protein) is a receptor protein, which contains the same or substantially the same amino acid sequence as the amino acid sequence shown by SEQ ID NO:1 (the amino acid sequences in FIGS. 1 and 2).

The receptor protein of the present invention may be any protein derived from any cells (e.g., retina cells, liver cells, splenocytes, nerve cells, glial cells, β cells of pancreas, bone marrow cells, mesangial cells, Langerhans' cells, epidermic cells, epithelial cells, endothelial cells, fibroblasts, fibrocytes, myocytes, fat cells, immune cells (e.g., macrophage, T cells, B cells, natural killer cells, mast cells, neutrophil, basophil, eosinophil, monocyte), megakaryocyte, synovial cells, chondrocytes, bone cells, osteoblasts, osteoclasts, mammary gland cells, hepatocytes or interstitial cells, the corresponding precursor cells, stem cells, cancer cells, etc.), hemocyte type cells, or any tissues where such cells are present, e.g., brain or any region of the brain (e.g., olfactory bulb, amygdaloid nucleus, basal ganglia, hippocampus, thalamus, hypothalamus, subthalamic nucleus, cerebral cortex, medulla oblongata, cerebellum, occipital pole, frontal lobe, temporal lobe, putamen, caudate nucleus, corpus callosum, substantia nigra), spinal cord, hypophysis, stomach, pancreas, kidney, liver, gonad, thyroid, gall-bladder, bone marrow, adrenal gland, skin, muscle, lung, gastrointestinal tract (e. g., large intestine and small intestine), blood vessel, heart, thymus, spleen, submandibular gland, peripheral blood, peripheral blood cells, prostate, testis, ovary, placenta, uterus, bone, joint, skeletal muscle, etc. (especially brain or any of brain regions) from human and other mammalians (e.g., guinea pigs, rats, mice, rabbits, swine, sheep, bovine, monkeys, etc.). The receptor protein may also be a synthetic protein.

The amino acid sequence which has substantially the same amino acid sequence as that represented by SEQ ID NO:1 includes an amino acid sequence having at least about 50% homology, preferably at least about 70% homology, more preferably at least about 80% homology, much more preferably at least about 90% homology, and most preferably at least about 95% homology, to the amino acid sequence represented by SEQ ID NO:1.

Examples of the protein which contains substantially the same amino acid sequence as that shown by SEQ ID NO:1 include a protein having substantially the same amino acid sequence as that shown by SEQ ID NO:1 and having the activity substantially equivalent to the amino acid sequence represented by SEQ ID NO:1, etc.

Examples of the substantially equivalent activity include a ligand binding activity, a signal transduction activity, etc. The term "substantially equivalent" is used to mean that the nature of the activity is the same. Therefore, although it is preferred that activities such as the ligand binding and signal transduction activities, etc. be equivalent (e.g., about 0.01- to about 100-fold, preferably about 0.5- to about 20-fold, more preferably about 0.5- to about 2-fold), quantitative factors such as a level of the activity, a molecular weight of the protein, etc. may differ.

The activities such as ligand binding and signal transduction activities or the like can be determined according to a publicly known method with some modifications, for example, by the ligand determination methods or the screening methods that will be later described.

Proteins containing the following amino acid sequences are used as the receptor protein of the present invention: ① amino acid sequences represented by SEQ ID NO:1, wherein at least 1 or 2 amino acids (preferably approximately 1 to 30 amino acids, more preferably approximately 1 to 10 amino acids, most preferably several (1 to 5) amino acids) are deleted; ② amino acid sequences represented by SEQ ID NO:1, to which at least 1 or 2 amino acids (preferably approximately 1 to 30 amino acids, more preferably approximately 1 to 10 amino acids, and most preferably several (1 to 5) amino acids) are added; ③ amino acid sequences represented by SEQ ID NO:1, in which at least 1 or 2 amino acids (preferably approximately 1 to 30 amino acids, more preferably approximately 1 to 10 amino acids, and most preferably several (1 to 5) amino acids) are substituted by other amino acids; or ④ combination of the amino acid sequences described in ① to ③ .

Throughout the present specification, the receptor proteins are represented in accordance with the conventional way of describing peptides, that is, the N-terminus (amino terminus) at the left hand and the C-terminus (carboxyl terminus) at the right hand. In the receptor proteins of the present invention including the receptor proteins containing the amino acid sequence shown by SEQ ID NO:1, the C-terminus is usually in the form of a carboxyl group (-COOH) or a carboxylate (-COO$^-$) but may be in the form of an amide (-CONH$_2$) or an ester (-COOR).

Examples of the ester group shown by R include a $C_{1-6}$ alkyl group such as methyl, ethyl, n-propyl, isopropyl, n-butyl, etc.; a $C_{3-8}$ cycloalkyl group such as cyclopentyl, cyclohexyl, etc.; a $C_{6-12}$ aryl group such as phenyl, α-naphthyl, etc.; a $C_{7-14}$ aralkyl group such as a phenyl-$C_{1-2}$-alkyl group, e.g., benzyl, phenethyl, etc., or an α-naphthyl-$C_{1-2}$-alkyl group such as α-naphthylmethyl, etc.; and the like. In addition, pivaloyloxymethyl or the like, which is used widely as an ester for oral administration, may also be used.

Where the receptor protein of the present invention contains a carboxyl group (or a carboxylate) at a position other than the C-terminus, it may be amidated or esterified and such an amide or ester is also included within the receptor protein of the present invention. The ester group may be the same group as that described with respect to the C-terminus described above.

Furthermore, examples of the receptor protein of the present invention include variants of the above receptor proteins, wherein the amino group at the N-terminal methionine residue of the protein supra is protected with a protecting group (for example, a $C_{1-6}$ acyl group such as a $C_{2-6}$ alkanoyl group, e.g., formyl group, acetyl group, etc.); those wherein the N-terminal region is cleaved in vivo and the glutamyl group thus formed is pyroglutaminated; those wherein a substituent (e.g., -OH, -SH, amino group, imidazole group, indole group, guanidino group, etc.) on the side chain of an amino acid in the molecule is protected with a suitable protecting group (e.g., a $C_{1-6}$ acyl group such as a $C_{2-6}$ alkanoyl group, e.g., formyl group, acetyl group, etc.), or conjugated proteins such as glycoproteins bound to sugar chains.

Specific examples of the receptor protein of the present invention which can be used include a human-derived (more preferably human hippocampus-derived) receptor protein, etc.

As partial peptides of the receptor protein of the present invention (hereinafter sometimes referred to as the partial peptides), any partial peptide can be used so long as it can be a partial peptide of the receptor protein. Among the receptor protein molecules of the present invention, for example, those having a site exposed to the outside of a cell membrane and having a receptor binding activity can be used.

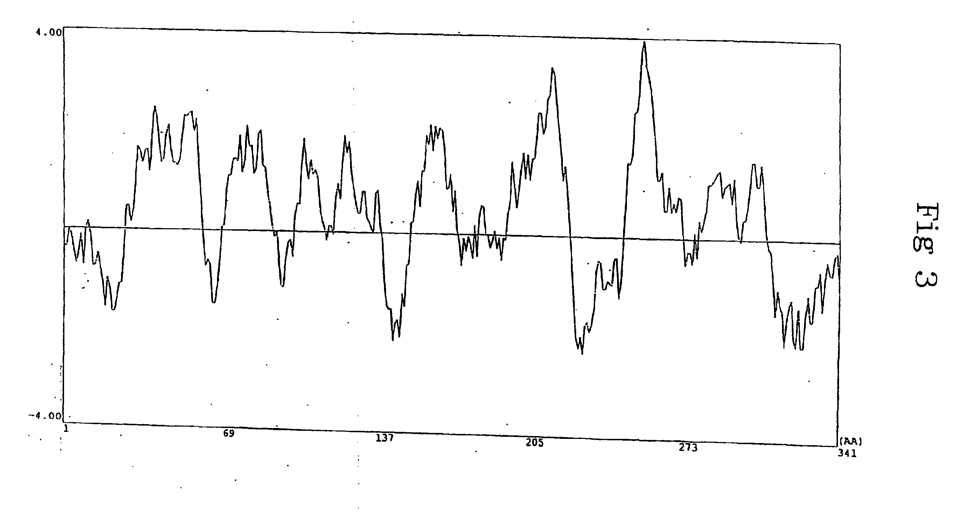

Specifically, the partial peptide of the receptor protein having the amino acid sequence represented by SEQ ID NO:1 is a peptide containing the parts analyzed to be extracellular domains (hydrophilic domains) in the hydrophobic plotting analysis, which is shown in FIG. 3. A peptide containing a hydrophobic domain in part can be used as well. In addition, the peptide may contain each domain separately or plural domains together.

In the receptor protein of the present invention, preferred partial peptides are those having at least 20, preferably at least 50, and more preferably at least 100 amino acids, in the amino acid sequence which constitutes the receptor protein of the present invention.

The amino acid sequence having substantially the same amino acid sequence includes an amino acid sequence having at least about 50% homology, preferably at least about 70% homology, more preferably at least about 80% homology, much more preferably at least about 90% homology, and most preferably at least about 95% homology, to these amino acid sequences.

Herein, the term "activity substantially equivalent" refers to the same significance as defined above.

The "activity substantially equivalent" can be assayed in the same manner as given above.

The partial peptide of the present invention may contain an amino acid sequence, wherein at least 1 or 2 amino acids (preferably approximately 1 to 10 amino acids, more preferably several (1 to 5) amino acids) are deleted; to which at least 1 or 2 amino acids (preferably approximately 1 to 20 amino acids, more preferably approximately 1 to 10 amino acids, and most preferably several (1 to 5) amino acids) are added; or, in which at least 1 or 2 amino acids (preferably approximately 1 to 10 amino acids, more preferably several and most preferably approximately 1 to 5 amino acids) are substituted by other amino acids.

In the partial peptide of the present invention, the C-terminus is normally a carboxyl group (-COOH) or carboxylate (-COO⁻) but the C-terminus may be in the form of an amide (-CONH$_2$) or an ester (-COOR), as has been described with the receptor protein of the present invention, wherein R has the same significance as defined above.

As in the receptor protein of the present invention described above, the partial peptide of the present invention further includes those in which the amino group of the amino acid residue of the N-terminal methionine residue is protected by a protecting group, those in which the N-terminal residue is cleaved in vivo and the produced glutamine residue is pyroglutaminated, those in which substituents on the side chains of amino acids in the molecule are protected by appropriate protecting groups, conjugated peptides such as so-called glycoproteins, to which sugar chains are bound, and the like.

In the partial peptide of the present invention, the C-terminus is normally a carboxyl group (-COOH) or carboxylate (-COO·) but the C-terminus may be in the form of an amide (-CONH$_2$) or an ester (-COOR), as has been described with the receptor protein of the present invention, wherein R has the same significance as defined above.

For salts of the receptor protein or the partial peptide of the present invention, preferred are salts with physiologically acceptable acids, especially physiologically acceptable acid addition salts. Examples of the salts include salts with, for example, inorganic acids (e. g., hydrochloric acid, phosphoric acid, hydrobromic acid, sulfuric acid); salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

The receptor protein of the present invention or salts thereof may be manufactured by a publicly known method used to purify a receptor protein from human or other mammalian cells or tissues described above, or by culturing a transformant transformed by the DNA encoding the receptor protein of the present invention, as will be later described. Furthermore, the receptor protein or its salts may also be manufactured by the methods for synthesizing proteins or by modifications thereof, which will also be described hereinafter.

Where the receptor protein or its salts are manufactured from human or mammalian tissues or cells, human or mammalian tissues or cells are homogenized, then extracted with an acid or the like, and the extract is isolated and purified by a combination of chromatography techniques such as reverse phase chromatography, ion exchange chromatography, and the like.

To synthesize the receptor protein of the present invention, its partial peptide, or salts or amides thereof according to the present invention, commercially available resins that are used for protein synthesis may be used. Examples of such resins include chloromethyl resin, hydroxymethyl resin, benzhydrylamine resin, aminomethy 1 resin, 4-benzyloxybenzyl alcohol resin, 4-methylbenzhydrylamine resin, PAM resin, 4-hydroxymethylmehtylphenyl acetamidomethyl resin, polyacrylamide resin, 4-(2',4'-dimethoxyphenylhydroxymethyl)phenoxy resin, 4-(2',4'-dimethoxyphenyl-Fmocaminoethyl) phenoxy resin, etc. Using these resins, amino acids in which $\alpha$-amino groups and functional groups on the side chains are appropriately protected are condensed on the resin in the order of the sequence of the objective protein according to various condensation methods publicly known in the art. At the end of the reaction, the receptor protein is cut out from the resin and at the same time, the protecting groups are removed. Then, intramolecular disulfide bond-forming reaction is performed in a highly diluted solution to obtain the objective protein or its amides.

For condensation of the protected amino acids described above, a variety of activation reagents for protein synthesis may be used, and carbodiimides are particularly preferable. Examples of such carbodiimides include DCC, N,N'-diisopropylcarbodiimide, N-ethyl-N'-(3-dimethylaminoprolyl)carbodiimide, etc. For activation by these reagents, the protected amino acids in combination with a racemization inhibitor (e.g., HOBt, HOOBt) are added directly to the resin, or the protected amino acids are previously activated in the form of symmetric acid anhydrides, HOBt esters or HOOBt esters, followed by adding the thus activated protected amino acids to the resin.

Solvents suitable for use to activate the protected amino acids or condense with the resin may be chosen from solvents known to be usable for protein condensation reactions. Examples of such solvents are acid amides such as N,N-dimethylformamide, N,N-dimethylacetamide, N-methylpyrrolidone, etc.; halogenated hydrocarbons such as methylene chloride, chloroform, etc.; alcohols such as trifluoroethanol, etc.; sulfoxides such as dimethylsulfoxide, etc.; ethers such as pyridine, dioxane, tetrahydrofuran, etc.; nitriles such as acetonitrile, propionitrile, etc.; esters such as methyl acetate, ethyl acetate, etc.; and appropriate mixtures of these solvents. The reaction temperature is appropriately chosen from the range known to be applicable to protein binding reactions and is usually selected in the range of approximately -20°C to 50°C. The activated amino acid derivatives are used generally in an excess of 1.5 to 4 times. The condensation is examined by a test using the ninhydrin reaction; when the condensation is insufficient, the condensation can be completed by repeating the condensation reaction without removal of the protecting groups. When the condensation is yet insufficient even after repeating the reaction, unreacted amino acids are acetylated with acetic anhydride or acetylimidazole.

Examples of the protecting groups used to protect the amino groups of the starting compounds include Z, Boc, t-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc, etc.

A carboxyl group can be protected by, e.g., alkyl esterification (in the form of linear, branched or cyclic alkyl esters of the alkyl moiety such as methyl, ethyl, propyl, butyl, t-butyl, cyclopentyl, cyclohexyl, cycloheptyl, cyclooctyl, 2-adamantyl, etc.), aralkyl esterification (e.g., esterification in the form of benzyl ester, 4-nitrobenzyl ester, 4-methoxybenzyl ester, 4-chlorobenzyl ester, benzhydryl ester, etc.), phenacyl esterification, benzyloxycarbonyl hydrazidation, t-butoxycarbonyl hydrazidation, trityl hydrazidation, or the like.

The hydroxyl group of serine can be protected through, for example, its esterification or etherification. Examples of groups appropriately used for the esterification include a lower alkanoyl group, such as acetyl group, an aroyl group such as benzoyl group, and a group derived from carbonic acid such as benzyloxycarbonyl group, ethoxycarbonyl group, etc. Examples of a group appropriately used for the etherification include benzyl group, tetrahydropyranyl group, t-butyl group, etc.

Examples of groups for protecting the phenolic hydroxyl group of tyrosine include Bzl, $Cl_2$-Bzl, 2-nitrobenzyl, Br-Z, t-butyl, etc.

Examples of groups used to protect the imidazole moiety of histidine include Tos, 4-methoxy-2,3,6-trimethylbenzenesulfonyl, DNP, benzyloxymethyl, Bum, Boc, Trt, Fmoc, etc.

Examples of the activated carboxyl groups in the starting compounds include the corresponding acid anhydrides, azides, activated esters (esters with alcohols (e.g., pentachlorophenol, 2,4,5-trichlorophenol, 2,4-dinitrophenol, cyanomethyl alcohol, p-nitrophenol, HONB, N-hydroxysuccimide, N-hydroxyphthalimide, HOBt)). As the activated amino acids, in which the amino groups are activated in the starting material, the corresponding phosphoric amides are employed.

To eliminate (split off) the protecting groups, there are used catalytic reduction under hydrogen gas flow in the presence of a catalyst such as Pd-black or Pd-carbon; an acid treatment with anhydrous hydrogen fluoride, methanesulfonic acid, trifluoromethanesulfonic acid or trifluoroacetic acid, or a mixture solution of these acids; a treatment with a base such as diisopropylethylamine, triethylamine, piperidine or piperazine; and reduction with sodium in liquid ammonia. The elimination of the protecting group by the acid treatment described above is carried out generally at a temperature of approximately -20°C to 40°C. In the acid treatment, it is efficient to add a cation scavenger such as anisole, phenol, thioanisole, m-cresol, p-cresol, dimethylsulfide, 1,4-butanedithiol or 1,2-ethanedithiol. Furthermore, 2,4-dinitrophenyl group known as the protecting group for the imidazole of histidine is removed by a treatment with thiophenol. Formyl group used as the protecting group of the indole of tryptophan is eliminated by the aforesaid acid treatment in the presence of 1,2-ethanedithiol or 1,4-butanedithiol, as well as by a treatment with an alkali such as a dilute sodium hydroxide solution and dilute ammonia.

Protection of functional groups that should not be involved in the reaction of the starting materials, protecting groups, elimination of the protecting groups and activation of functional groups involved in the reaction may be appropriately selected from publicly known groups and publicly known means.

In another method for obtaining the amides of the protein, for example, the $\alpha$-carboxyl group of the carboxy terminal amino acid is first protected by amidation; the peptide (protein) chain is then extended from the amino group side to a desired length. Thereafter, a protein in which only the protecting group of the N-terminal $\alpha$-amino group in the peptide chain has been eliminated from the protein and a protein in which only the protecting group of the C-terminal carboxyl group has been eliminated are prepared. The two proteins are condensed in a mixture of the solvents described above. The details of the condensation reaction are the same as described above. After the protected protein obtained by the condensation is purified, all the protecting groups are eliminated by the method described above to give the desired crude protein. This crude protein is purified by various known purification means. Lyophilization of the major fraction gives the amide of the desired protein.

To prepare the esterified protein, for example, the $\alpha$-carboxyl group of the carboxy terminal amino acid is condensed with a desired alcohol to prepare the amino acid ester, which is followed by procedure similar to the preparation of the amidated protein above to give the ester form of the desired protein.

The partial peptide or its salts in the protein of the present invention can be manufactured by publicly known methods for peptide synthesis, or by cleaving the protein of the present invention with an appropriate peptidase. For the methods for peptide synthesis, for example, either solid phase synthesis or liquid phase synthesis may be used. That is, the partial peptide or amino acids that can construct the protein of the present invention are condensed with the remaining part. Where the product contains protecting groups, these protecting groups are removed to give the desired peptide. Publicly known methods for condensation and elimination of the protecting groups are described in 1) - 5) below.

1) M. Bodanszky & M.A. Ondetti: Peptide Synthesis, Interscience Publishers, New York (1966)
2) Schroeder & Luebke: The Peptide, Academic Press, New York (1965)
3) Nobuo Izumiya, et al.: *Peptide Gosei-no-Kiso to Jikken* (Basics and experiments of peptide synthesis), published by Maruzen Co. (1975)
4) Haruaki Yajima & Shunpei Sakakibara: *Seikagaku Jikken Koza* (Biochemical Experiment) 1, *Tanpakushitsu no Kagaku* (Chemistry of Proteins) IV, 205 (1977)
5) Haruaki Yajima, ed.: *Zoku Iyakuhin no Kaihatsu* (A sequel to Development of Pharmaceuticals), Vol. 14, Peptide Synthesis, published by Hirokawa Shoten

After completion of the reaction, the product may be purified and isolated by a combination of conventional purification methods such as solvent extraction, distillation, column chromatography, liquid chromatography and recrystallization to give the partial peptide of the present invention. When the partial peptide obtained by the above methods is in a free form, the peptide can be converted into an appropriate salt by a publicly known method; when the protein is obtained in a salt form, it can be converted into a free form by a publicly known method.

The polynucleotide encoding the receptor protein of the present invention may be any polynucleotide so long as it contains the base sequence (DNA or RNA, preferably DNA) encoding the receptor protein of the present invention described above. Such a polynucleotide may also be any one of DNA encoding the receptor protein of the present invention, RNA such as mRNA, etc., and may be double-stranded or single-stranded. Where the polynucleotide is double-stranded, it may be double-stranded DNA, double-stranded RNA or DNA: RNA hybrid. Where the polynucleotide is single-stranded, it may be a sense strand (i.e., a coding strand) or an antisense strand (i.e., a non-coding strand).

Using the polynucleotide encoding the receptor protein of the present invention, mRNA of the receptor protein of the present invention can be quantified by, for example, the publicly known method published in separate volume of *Jikken Igaku* 15 (7) "New PCR and its application" (1997), or by its modifications.

The DNA encoding the receptor protein of the present invention may be any of genomic DNA, genomic DNA library, cDNA derived from the cells and tissues described above, cDNA library derived from the cells and tissues described above and synthetic DNA. The vector to be used for the library may be any of bacteriophage, plasmid, cosmid and phagemid. The DNA may also be directly amplified by reverse transcriptase polymerase chain reaction (hereinafter abbreviated as RT-PCR) using the total RNA or mRNA fraction prepared from the cells and tissues described above.

Specifically, the DNA encoding the receptor protein of the present invention may be any DNA having the base sequence shown by SEQ ID NO:2 or the base sequence hybridizable to the base sequence represented by SEQ ID NO:2 under highly stringent conditions and encoding a receptor protein having the activities substantially equivalent to those of the receptor protein of the present invention (e.g., a ligand binding activity, a signal transduction activity, etc.).

Specific examples of the DNA hybridizable to the base sequence represented by SEQ ID NO:2 include DNA containing a base sequence having at least about 70% homology, preferably at least about 80% homology, more preferably at least about 90% homology and most preferably at least about 95% homology, to the base sequence represented by SEQ ID NO:2.

The hybridization can be carried out by publicly known methods or by modifications of these methods, for example, according to the method described in Molecular Cloning, 2nd (J. Sambrook et al., Cold Spring Harbor Lab. Press, 1989). A commercially available library may also be used according to the instructions of the attached manufacturer's protocol. Preferably, the hybridization can be carried out under highly stringent conditions.

The highly stringent conditions used herein are, for example, those in a sodium concentration at about 19 mM to about 40 mM, preferably about 19 mM to about 20 mM at a temperature of about 50°C to about 70°C, preferably about 60°C to about 65°C. In particular, hybridization conditions in a sodium concentration of about 19 mM at a temperature of about 65°C are most preferred.

More specifically, for the DNA encoding the receptor protein having the amino acid sequence represented by SEQ ID NO:1, there may be employed DNA having the base sequence represented by SEQ ID NO: 2.

The polynucleotide comprising a part of the base sequence of the DNA encoding the receptor protein of the present invention or a part of the base sequence complementary to the DNA is used to mean to embrace not only the DNA encoding the partial peptide of the present invention described below but also RNA.

According to the present invention, antisense polynucleotides (nucleic acids) that can inhibit the replication or expression of G protein-coupled receptor protein genes can be designed and synthesized based on the base sequence information of the cloned or determined DNA encoding the G protein-coupled receptor protein. Such a polynucleotide (nucleic acid) is capable of hybridizing to RNA of G protein-coupled receptor protein gene to inhibit the synthesis or function of said RNA or capable of modulating or controlling the expression of a G protein-coupled receptor protein gene via interaction with G protein-coupled receptor protein-associated RNA. Polynucleotides complementary to the selected sequences of RNA associated with G protein-coupled receptor protein and polynucleotides specifically hybridizable to the G protein-coupled receptor protein-associated RNA are useful in modulating or controlling the expression of a G protein-coupled receptor protein gene in vivo and in vitro, and useful for the treatment or diagnosis of diseases. The term "corresponding" is used to mean homologous to or complementary to a particular sequence of the nucleotide, base sequence or nucleic acid including the gene. The term "corresponding" between nucleotides, base sequences or nucleic acids and peptides (proteins) usually refers to amino acids of a peptide (protein) under the order derived from the sequence of nucleotides (nucleic acids) or their complements. In the G protein-coupled receptor protein genes, the 5' end hairpin loop, 5' end 6-base-pair repeats, 5' end untranslated region, polypeptide translation initiation codon, protein coding region, ORF translation initiation codon, 3' end untranslated region, 3' end palindrome region, and 3' end hairpin loop, may be selected as preferred target regions, though any other region may be selected as a target in the G protein-coupled receptor protein genes.

The relationship between the targeted nucleic acids and the polynucleotides complementary to at least a part of the target, specifically the relationship between the target and the polynucleotides hybridizable to the target, can be denoted to be "antisense". Examples of the antisense polynucleotides include polydeoxynucleotides containing 2-deoxy-D-ribose, polydeoxynucleotides containing D-ribose, any other type of polynucleotides which are N-glycosides of a purine or pyrimidine base, or other polymers containing non-nucleotide backbones (e.g., protein nucleic acids and synthetic sequence-specific nucleic acid polymers commercially available) or other polymers containing nonstandard linkages (provided that the polymers contain nucleotides having such a configuration that allows base pairing or base stacking, as is found in DNA or RNA), etc. The antisense polynucleotides may be double-stranded DNA, single-stranded DNA, single-stranded RNA or a DNA:RNA hybrid, and may further include unmodified polynucleotides (or unmodified oligonucleotides), those with publicly known types of modifications, for example, those with labels known in the art, those with caps, methylated polynucleotides, those with substitution of one or more naturally occurring nucleotides by their analogue, those with intramolecular modifications of nucleotides such as those with uncharged linkages (e.g., methyl phosphonates, phosphotriesters, phosphoramidates, carbamates, etc.) and those with charged linkages or sulfur-containing linkages (e.g., phosphorothioates, phosphorodithioates, etc.), those having side chain groups such as proteins (nucleases, nuclease inhibitors, toxins, antibodies, signal peptides, poly-L-lysine, etc.), saccharides (e.g., monosaccharides, etc.), those with intercalators (e.g., acridine, psoralen, etc.), those containing chelators (e.g., metals, radioactive metals, boron, oxidative metals, etc.), those containing alkylating agents, those with modified linkages (e.g., $\alpha$ anomeric nucleic acids, etc.), and the like. Herein the terms "nucleoside", "nucleotide" and "nucleic acid" are used to refer to moieties that contain not only the purine and pyrimidine bases, but also other heterocyclic bases, which have been modified. Such modifications may include methylated purines and pyrimidines, acylated purines and pyrimidines and other heterocyclic rings. Modified nucleotides and modified nucleotides also include modifications on the sugar moiety, wherein, for example, one or more hydroxyl groups may optionally be substituted with a halogen atom(s), an aliphatic group(s), etc., or may be converted into the corresponding functional groups such as ethers, amines, or the like.

The antisense polynucleotide (nucleic acid) of the present invention is RNA, DNA or a modified nucleic acid (RNA, DNA). Specific examples of the modified nucleic acid are, but not limited to, sulfur and thiophosphate derivatives of nucleic acids and those resistant to degradation of polynucleoside amides or oligonucleoside amides. The antisense nucleic acids of the present invention can be modified preferably based on the following design, that is, by increasing the intracellular stability of the antisense nucleic acid, increasing the cellular permeability of the antisense nucleic acid, increasing the affinity of the nucleic acid to the targeted sense strand to a higher level, or minimizing the toxicity, if any, of the antisense nucleic acid.

Many of such modifications are known in the art, as disclosed in J. Kawakami, et al., Pharm. Tech. Japan, Vol. 8, pp. 247, 1992; Vol. 8, pp. 395, 1992; S. T. Crooke, et al. ed., Antisense Research and Applications, CRC Press, 1993; etc.

The antisense nucleic acid of the present invention may contain altered or modified sugars, bases or linkages. The antisense nucleic acid may also be provided in a specialized form such as liposomes, microspheres, or may be applied to gene therapy, or may be provided in combination with attached moieties. Such attached moieties include polycations such as polylysine that act as charge neutralizers of the phosphate backbone, or hydrophobic moieties such as lipids (e.g., phospholipids, cholesterols, etc.) that enhance the interaction with cell membranes or increase uptake of the nucleic acid. Preferred examples of the lipids to be attached are cholesterols or derivatives thereof (e.g., cholesteryl chloroformate, cholic acid, etc.). These moieties may be attached to the nucleic acid at the 3' or 5' ends thereof and may also be attached thereto through a base, sugar, or intramolecular nucleoside linkage. Other moieties may be capping groups specifically placed at the 3' or 5' ends of the nucleic acid to prevent degradation by nucleases such as exonuclease, RNase, etc. Such capping groups include, but are not limited to, hydroxyl protecting groups known in the art, including glycols such as polyethylene glycol, tetraethylene glycol and the like.

The inhibitory action of the antisense nucleic acid can be examined using the transformant of the present invention, the gene expression system of the present invention in vivo and in vitro, or the translation system of the G protein-coupled receptor protein in vivo and in vitro. The nucleic acid can be applied to cells by a variety of publicly known methods.

The DNA encoding the partial peptide of the present invention may be any DNA so long as it contains the base sequence encoding the partial peptide of the present invention described above. The DNA may also be any of genomic DNA, genomic DNA library, cDNA derived from the cells and tissues described above, cDNA library derived from the cells and tissues described above and synthetic DNA. The vector to be used for the library may be any of bacteriophage, plasmid, cosmid and phagemid. The DNA may also be directly amplified by reverse transcriptase polymerase chain reaction (hereinafter abbreviated as RT-PCR) using mRNA fraction prepared from the cells and tissues described above.

Specifically, the DNA encoding the partial peptide of the present invention may be any one of, for example, (1) DNA containing a partial base sequence of the DNA having the base sequence represented by SEQ ID NO:2, or (2) any DNA containing a partial base sequence of the DNA having a base sequence hybridizable to the base sequence represented by SEQ ID NO: 2 under highly stringent conditions and encoding a receptor protein which has the activities (e.g., a ligand-biding activity, a signal transduction activity, etc.) substantially equivalent to those of the receptor protein peptide of the present invention.

Specific examples of the DNA that is hybridizable to the base sequence represented by SEQ ID NO: 2 include DNA containing a base sequence having at least about 70% homology, preferably at least about 80% homology, more preferably at least about 90% homology and most preferably at least about 95% homology, to the base sequence represented by SEQ ID NO: 2.

For cloning of the DNA that completely encodes the receptor protein of the present invention or its partial peptide (hereinafter sometimes collectively referred to as the receptor protein of the present invention), the DNA may be either amplified by PCR using synthetic DNA primers containing a part of the base sequence of the receptor protein of the present invention, or the DNA inserted into an appropriate vector can be selected by hybridization with a labeled DNA fragment or synthetic DNA that encodes a part or entire region of the receptor protein of the present invention. The hybridization can be carried out, for example, according to the method described in Molecular Cloning, 2nd, J. Sambrook et al., Cold Spring Harbor Lab. Press, 1989. The hybridization may also be performed using commercially available library in accordance with the protocol described in the attached instructions.

Conversion of the base sequence of the DNA can be effected by publicly known methods such as the Gupped duplex method or the Kunkel method or its modification by using a publicly known kit available as Mutan™-G or Mutan™-K (both manufactured by Takara Shuzo Co., Ltd.).

The cloned DNA encoding the receptor protein can be used as it is, depending upon purpose or, if desired, after digestion with a restriction enzyme or after addition of a linker thereto. The DNA may contain ATG as a translation initiation codon at the 5' end thereof and may further contain TAA, TGA or TAG as a translation termination codon at the 3' end thereof. These translation initiation and termination codons may also be added by using an appropriate synthetic DNA adapter.

The expression vector for the receptor protein of the present invention can be manufactured, for example, by (a) excising the desired DNA fragment from the DNA encoding the receptor protein of the present invention, and then (b) ligating the DNA fragment with an appropriate expression vector downstream a promoter in the vector.

Examples of the vector include plasmids derived form E. coli (e.g., pBR322, pBR325, pUC12, pUC13), plasmids derived from Bacillus subtilis (e.g., pUB110, pTP5, pC194), plasmids derived from yeast (e. g., pSH19, pSH15), bacteriophages such as λ phage, etc., animal viruses such as retrovirus, vaccinia virus, baculovirus, etc. as well as pA1-11, pXT1, pRc/CMV, pRc/RSV, pcDNAI/Neo, etc.

The promoter used in the present invention may be any promoter if it matches well with a host to be used for gene expression. In the case of using animal cells as the host, examples of the promoter include SRα promoter, SV40 promoter, LTR promoter, CMV promoter, HSV-TK promoter, etc.

Among them, CMV promoter or SRα promoter is preferably used. Where the host is bacteria of the genus Escherichia, preferred examples of the promoter include trp promoter, lac promoter, recA promoter, λP$_L$ promoter, lpp promoter, etc. In the case of using bacteria of the genus Bacillus as the host, preferred example of the promoter are SPO1 promoter, SPO2 promoter and penP promoter. When yeast is used as the host, preferred examples of the promoter are PHO5 promoter, PGK promoter, GAP promoter and ADH promoter. When insect cells are used as the host, preferred examples of the promoter include polyhedrin prompter and P10 promoter.

In addition to the foregoing examples, the expression vector may further optionally contain an enhancer, a splicing signal, a poly A addition signal, a selection marker, SV40 replication origin (hereinafter sometimes abbreviated as SV40ori) etc. Examples of the selection marker include dihydrofolate reductase (hereinafter sometimes abbreviated as dhfr) gene [methotrexate (MTX) resistance], ampicillin resistant gene (hereinafter sometimes abbreviated as Amp$^r$), neomycin resistant gene (hereinafter sometimes abbreviated as Neo$^r$, G418 resistance), etc. In particular, when dhfr gene is used as the selection marker in CHO (dhfr·) cells, selection can also be made on thymidine free media.

If necessary and desired, a signal sequence that matches with a host is added to the N-terminus of the receptor protein of the present invention. Examples of the signal sequence that can be used are Pho A signal sequence, OmpA signal sequence, etc. in the case of using bacteria of the genus Escherichia as the host; α-amylase signal sequence, subtilisin signal sequence, etc. in the case of using bacteria of the genus Bacillus as the host; MFα signal sequence, SUC2 signal sequence, etc. in the case of using yeast as the host; and insulin signal sequence, α-interferon signal sequence, antibody molecule signal sequence, etc. in the case of using animal cells as the host, respectively.

Using the vector containing the DNA encoding the receptor protein of the present invention thus constructed, transformants can be manufactured.

Examples of the host, which may be employed, are bacteria belonging to the genus Escherichia, bacteria belonging to the genus Bacillus, yeast, insect cells, insects and animal cells, etc.

Specific examples of the bacteria belonging to the genus Escherichia include Escherichia coli K12 DH1 (Proc. Natl. Acad. Sci. U.S.A., 60, 160 (1968)), JM103 (Nucleic Acids Research, 9, 309 (1981)), JA221 (Journal of Molecular Biology, 120, 517 (1978)), HB101 (Journal of Molecular Biology, 41, 459 (1969)), C600 (Genetics, 39, 440 (1954)), etc.

Examples of the bacteria belonging to the genus Bacillus include Bacillus subtilis MI114 (Gene, 24, 255 (1983)), 207-21 (Journal of Biochemistry, 95, 87 (1984)), etc.

Examples of yeast include Saccharomyces cereviseae AH22, AH22R⁻, NA87-11A, DKD-5D, 20B-12,
Schizosaccharomyces pombe NCYC1913, NCYC2036, Pichia pastoris KM71, etc.

Examples of insect cells include, for the virus AcNPV, Spodoptera frugiperda cells (Sf cells), MG1 cells derived from mid-intestine of Trichoplusia ni, High Five™ cells derived from egg of Trichoplusia ni, cells derived from Mamestra brassicae, cells derived from Estigmena acrea, etc.; and for the virus BmNPV, Bombyx mori N cells (BmN cells), etc. are used. Examples of the Sf cell which can be used are Sf9 cells (ATCC CRL1711) and Sf21 cells (both cells are described in Vaughn, J. L. et al., In Vivo, 13, 213-217 (1977).

As the insect, for example, a larva of Bombyx mori can be used (Maeda, et al., Nature, 315, 592 (1985)).

Examples of animal cells include monkey cells COS-7, Vero, Chinese hamster cells CHO (hereinafter referred to as CHO cells), dhfr gene deficient Chinese hamster cells CHO (hereinafter simply referred to as CHO(dhfr) cell), mouse L cells, mouse AtT-20, mouse myeloma cells, rat GH3, human FL cells, etc.

Bacteria belonging to the genus Escherichia can be transformed, for example, by the method described in Proc. Natl. Acad. Sci. U.S.A., 69, 2110 (1972) or Gene, 17, 107 (1982). Bacteria belonging to the genus Bacillus can be transformed, for example, by the method described in Molecular & General Genetics, 168, 111 (1979).

Yeast can be transformed, for example, by the method described in Methods in Enzymology, 194, 182-187 (1991), Proc. Natl. Acad. Sci. U.S.A., 75, 1929 (1978), etc.

Insect cells or insects can be transformed, for example, according to the method described in Bio/Technology, 6, 47-55(1988), etc.

Animal cells can be transformed, for example, according to the method described in *Saibo Kogaku* (Cell Engineering), extra issue 8, *Shin Saiba Kogaku Jikken Protocol* (New Cell Engineering Experimental Protocol), 263-267 (1995), published by Shujunsha, or Virology, 52, 456 (1973).

Thus, the transformant transformed with the expression vector containing the DNA encoding the G protein-coupled receptor protein can be obtained.

Where the host is bacteria belonging to the genus Escherichia or the genus Bacillus, the transformant can be appropriately incubated in a liquid medium which contains materials required for growth of the transformant such as carbon sources, nitrogen sources, inorganic materials, and so on. Examples of the carbon sources include glucose, dextrin, soluble starch, sucrose, etc. Examples of the nitrogen sources include inorganic or organic materials such as ammonium salts, nitrate salts, corn steep liquor, peptone, casein, meat extract, soybean cake, potato extract, etc. Examples of the inorganic materials are calcium chloride, sodium dihydrogenphosphate, magnesium chloride, etc. In addition, yeast, vitamins, growth promoting factors etc. may also be added to the medium. Preferably, pH of the medium is adjusted to about 5 to about 8.

A preferred example of the medium for incubation of the bacteria belonging to the genus Escherichia is M9 medium supplemented with glucose and Casamino acids (Miller, Journal of Experiments in Molecular Genetics, 431-433, Cold Spring Harbor Laboratory, New York, 1972). If necessary and desired, a chemical such as 3β-indolylacrylic acid can be added to the medium thereby to activate the promoter efficiently. Where the bacteria belonging to the genus Escherichia are used as the host, the transformant is usually cultivated at about 15°C to about 43°C for about 3 hours to about 24 hours. If necessary and desired, the culture may be aerated or agitated.

Where the bacteria belonging to the genus Bacillus 'are used as the host, the transformant is cultivated generally at about 30°C to about 40°C for about 6 hours to about 24 hours. If necessary and desired, the culture can be aerated or agitated.

Where yeast is used as the host, the transformant is cultivated, for example, in Burkholder's minimal medium (Bostian, K. L. et al., Proc. Natl. Acad. Sci. U.S.A., 77, 4505 (1980)) or in SD medium supplemented with 0.5% Casamino acids (Bitter, G. A. et al., Proc. Natl. Acad. Sci. U.S.A., 81, 5330 (1984)). Preferably, pH of the medium is adjusted to about 5 to about 8. In general, the transformant is cultivated at about 20°C to about 35°C for about 24 hours to about 72 hours. If necessary and desired, the culture can be aerated or agitated.

Where insect cells or insects are used as the host, the transformant is cultivated in, for example, Grace's Insect Medium (Grace, T. C. C., Nature, 195, 788 (1962)) to which an appropriate additive such as immobilized 10% bovine serum is added. Preferably, pH of the medium is adjusted to about 6.2 to about 6.4. Normally, the transformant is cultivated at about 27°C for about 3 days to about 5 days and, if necessary and desired, the culture can be aerated or agitated.

Where animal cells are employed as the host, the transformant is cultivated in, for example, MEM medium containing about 5% to about 20% fetal bovine serum (Science, 122, 501 (1952)), DMEM medium (Virology, 8, 396 (1959)), RPMI 1640 medium (The Journal of the American Medical Association, 199, 519 (1967)), 199 medium (Proceeding of the Society for the Biological Medicine, 73, 1 (1950)), etc. Preferably, pH of the medium is adjusted to about 6 to about 8. The transformant is usually cultivated at about 30°C to about 40°C for about 15 hours to about 60 hours and, if necessary and desired, the culture can be aerated or agitated.

As described above, the G protein-coupled receptor protein of the present invention can be produced in the cell membrane of the transformant, etc.

The receptor protein of the present invention can be separated and purified from the culture described above by the following procedures.

When the receptor protein of the present invention is extracted from the culture or cells, after cultivation the transformants or cells are collected by a publicly known method and suspended in a appropriate buffer. The transformants or cells are then disrupted by publicly known methods such as ultrasonication, a treatment with lysozyme and/or freeze-thaw cycling, followed by centrifugation, filtration, etc. Thus, the crude extract of the receptor protein of the present invention can be obtained. The buffer used for the procedures may contain a protein modifier such as urea or guanidine hydrochloride, or a surfactant such as Triton X-100™, etc. When the receptor protein is secreted in the culture, after completion of the cultivation the supernatant can be separated from the transformants or cells to collect the supernatant by a publicly known method.

The receptor protein contained in the supernatant or the extract thus obtained can be purified by appropriately combining the publicly known methods for separation and purification. Such publicly known methods for separation and purification include a method utilizing difference in solubility such as salting out, solvent precipitation, etc.; a method utilizing mainly difference in molecular weight such as dialysis, ultrafiltration, gel filtration, SDS-polyacrylamide gel electrophoresis, etc.; a method utilizing difference in electric charge such as ion exchange chromatography, etc.; a method utilizing difference in specific affinity such as affinity chromatography, etc.; a method utilizing difference in hydrophobicity such as reverse phase high performance liquid chromatography, etc.; a method utilizing difference in isoelectric point such as isoelectrofocusing electrophoresis; and the like.

When the receptor protein thus obtained is in a free form, it can be converted into the salt by publicly known methods or modifications thereof. On the other hand, when the receptor protein is obtained in the form of a salt, it can be converted into the free form or in the form of a different salt by publicly known methods or modifications thereof.

The receptor protein produced by the recombinant can be treated, prior to or after the purification, with an appropriate protein modifying enzyme so that the receptor protein can be appropriately modified to partially remove a polypeptide. Examples of the protein-modifying enzyme include trypsin, chymotrypsin, arginyl endopeptidase, protein kinase, glycosidase or the like.

The activity of the thus produced receptor protein of the present invention or salts thereof can be determined by a test binding to a labeled ligand, by an enzyme immunoassay using a specific antibody, or the like.

Antibodies to the receptor protein of the present invention, its partial peptides, or salts thereof may be any of polyclonal antibodies and monoclonal antibodies, as long as they are capable of recognizing the receptor protein of the present invention, its partial peptides, or salts thereof.

The antibodies to the receptor protein of the present invention, its partial peptides, or salts thereof (hereinafter sometimes merely referred to as the receptor protein of the present invention) may be manufactured by publicly known methods for manufacturing antibodies or antisera, using as antigens the receptor protein of the present invention.

[Preparation of monoclonal antibody]

(a) Preparation of monoclonal antibody-producing cells

The receptor protein of the present invention is administered to mammals either solely or together with carriers or diluents to the site where the production of antibody is possible by the administration. In order to potentiate the antibody productivity upon the administration, complete Freund's adjuvants or incomplete Freund's adjuvants may be administered. The administration is usually carried out once in every two to six weeks and 2 to 10 times in total. Examples of the applicable mammals are monkeys, rabbits, dogs, guinea pigs, mice, rats, sheep and goats, with mice and rats being preferred.

In the preparation of monoclonal antibody-producing cells, warm-blooded animals, e.g., mice, immunized with an antigen wherein the antibody titer is noted is selected, then the spleen or lymph node is collected after 2 to 5 days from the final immunization and antibody-producing cells contained therein are fused with myeloma cells to give monoclonal antibody-producing hybridomas. Measurement of the antibody titer in antisera may be made, for example, by reacting a labeled form of the receptor protein, which will be described later, with the antiserum followed by assaying the binding activity of the labeling agent bound to the antibody. The fusion may be operated, for example, by the known Koehler and Milstein method (Nature, 256, 495, 1975). Examples of the fusion accelerator are polyethylene glycol (PEG), Sendai virus, etc., of which PEG is preferably employed.

Examples of the myeloma cells are NS-1, P3U1, SP2/0, etc. In particular, P3U1 is preferably employed. A preferred ratio of the count of the antibody-producing cells used (spleen cells) to the count of myeloma cells is within a range of approximately 1:1 to 20:1. When PEG (preferably, PEG 1000 to PEG 6000) is added in a concentration of approximately 10 to 80% followed by incubating at about 20 to about 40°C, preferably at about 30 to about 37°C for about 1 to about 10 minutes, an efficient cell fusion can be carried out.

Various methods can be used for screening of a monoclonal antibody-producing hybridoma. Examples of such methods include a method which comprises adding the supernatant of hybridoma to a solid phase (e.g., microplate) adsorbed with the receptor protein of the present invention as an antigen directly or together with a carrier, adding an anti-immunoglobulin antibody (when mouse cells are used for the cell fusion, antimouse immunoglobulin antibody is used) labeled with a radioactive substance or an enzyme, or Protein A and detecting the monoclonal antibody bound to the solid phase, and a method which comprises adding the supernatant of hybridoma to a solid phase adsorbed with an anti-immunoglobulin antibody or Protein A, adding the receptor protein labeled with a radioactive substance or an enzyme and detecting the monoclonal antibody bound to the solid phase.

The monoclonal antibody can be selected by publicly known methods or by modifications of these methods. In general, the selection can be effected in a medium for animal cells supplemented with HAT (hypoxanthine, aminopterin and thymidine). Any selection and growth medium can be employed as far as the hybridoma can grow therein. For example, RPMI 1640 medium containing 1% to 20%, preferably 10% to 20% fetal bovine serum, GIT medium (Wako Pure Chemical Industries, Ltd.) containing 1% to 10% fetal bovine serum, a serum free medium for cultivation of a hybridoma (SFM-101, Nissui Seiyaku Co., Ltd.) and the like can be used for the selection and growth medium. The cultivation is carried out generally at 20°C to 40°C, preferably at about 37°C, for 5 days to 3 weeks, preferably 1 to 2 weeks. The cultivation can be conducted normally in 5% $CO_2$. The antibody titer of the culture supernatant of hybridomas can be determined as in the assay for the antibody titer in antisera described above.

(b) Purification of monoclonal antibody

Separation and purification of a monoclonal antibody can be carried out by methods applied to conventional separation and purification of immunoglobulins, as in the conventional methods for separation and purification of polyclonal antibodies [e.g., salting-out, alcohol precipitation, isoelectric point precipitation, electrophoresis, adsorption and desorption with ion exchangers (e.g., DEAE), ultracentrifugation, gel filtration, or a specific purification method which comprises collecting only an antibody with an activated adsorbent such as an antigen-binding solid phase, Protein A, Protein G, etc. and dissociating the binding to obtain the antibody].

[Preparation of polyclonal antibody]

The polyclonal antibody of the present invention can be manufactured by publicly known methods or modifications thereof. For example, a complex of immunogen (receptor protein antigen) and a carrier protein is prepared, and a mammal is immunized with the complex in a manner similar to the method described above for the manufacture of monoclonal antibodies. The product containing the antibody to the receptor protein of the present invention is collected from the immunized animal followed by separation and purification of the antibody.

In the complex of an immunogen and a carrier protein used to immunize a mammal, the type of carrier protein and the mixing ratio of a carrier to hapten may be any type and in any ratio, as long as the antibody is efficiently produced to the hapten immunized by crosslinking to the carrier. For example, bovine serum albumin, bovine thyroglobulins, keyhole limpet hemocyanin, etc. is coupled to hapten in a carrier-to-hapten weight ratio of approximately 0.1 to 20, preferably about 1 to about 5.

A variety of condensing agents can be used for the coupling of a carrier to hapten. Glutaraldehyde, carbodiimide, maleimide activated ester, activated ester reagents containing thiol group or dithiopyridyl group, etc. are used for the coupling.

The condensation product is administered to warm-blooded animals either solely or together with carriers or diluents to the site in which the antibody can be produce by the administration. In order to potentiate the antibody productivity upon the administration, complete Freund's adjuvant or incomplete Freund's adjuvant may be administered. The administration is usually made once approximately in every 2 to 6 weeks and about 3 to about 10 times in total.

The polyclonal antibody can be collected from the blood, ascites, etc., preferably from the blood of mammals immunized by the method described above.

The polyclonal antibody titer in antiserum can be assayed by the same procedure as that for the determination of serum antibody titer described above. The separation and purification of the polyclonal antibody can be carried out, following the method for the separation and purification of immunoglobulins performed as applied to the separation and purification of monoclonal antibodies described hereinabove.

The receptor protein of the present invention, its salts, its partial peptides, or salts thereof, and the DNA encoding the receptor protein or the partial peptide can be used for: (1) determination of ligands (agonists) to the G protein-coupled receptor protein of the present invention, (2) prophylactic and/or therapeutic agents for diseases associated with dysfunction of the G protein-coupled receptor protein of the present invention, (3) agents for gene diagnosis, (4) methods of screening compounds that alter the expression level of the receptor protein of the present invention or its partial peptides, (5) prophylactic and/or therapeutic agents for various diseases comprising a compound that alters the expression level of the receptor protein of the present invention or its partial peptides, (6) methods of quantification of ligands to the G protein-coupled receptor protein of the present invention, (7) methods of screening compounds (agonists, antagonists, etc.) that alter the binding property between the G protein-coupled receptor protein of the present invention and ligands, (8) prophylactic and/or therapeutic agents for various diseases comprising a compound (an agonist or an antagonist) that alters the binding property between the G protein-coupled receptor protein of the present invention and ligands, (9) quantification of the receptor protein of the present invention, its partial peptides or salts thereof, (10) methods of screening compounds that alter the amount of the receptor protein of the present invention or its partial peptides in cell membranes, (11) prophylactic and/or therapeutic agents for various diseases comprising a compound that alters the amount of the receptor protein of the present invention or its partial peptides in cell membranes, (12) neutralization by antibodies to the receptor protein of the present invention, its partial peptides, or salts thereof, and (13) preparation of non-human animals that possess the DNA encoding the G protein-coupled receptor protein of the present invention.

In particular, by the use of the receptor binding assay system using the expression system of the recombinant G protein-coupled receptor protein of the present invention, compounds (e.g., agonists, antagonists, etc.) that alter the binding property of human- or mammal-specific ligands for the G protein-coupled receptor protein can be screened, and the agonists or antagonists can be used as prophylactic and therapeutic agents for various diseases.

Hereinafter, the receptor protein of the present invention, its partial peptides, or salts thereof (hereinafter sometimes referred to as the receptor protein of the present invention), the DNA encoding the receptor protein of the present invention or its partial peptides (hereinafter sometimes referred to as the DNA of the present invention) and the antibodies to the receptor protein of the present invention (hereinafter sometimes referred to as the antibodies of the present invention) are specifically described for the use or applications.

(1) Determination of a ligand (agonist) to the G protein-coupled receptor protein of the present invention

The receptor protein of the present invention or its salts, or the partial peptide or its salts of the present invention are useful as reagents for searching and determining ligands (agonists) to the receptor protein of the present invention or its salts.

That is, the present invention provides a method for determining a ligand to the receptor protein of the present invention, which comprises bringing the receptor protein of the present invention or its salts, or the partial peptide of the present invention or its salts, in contact with a test compound.

Examples of the test compound include publicly known ligands (e.g., angiotensin, bombesin, canavinoid, cholecystokinin, glutamine, serotonin, melatonin, neuropeptide Y, opioid, purines, vasopressin, oxytocin, PACAP, secretin, glucagon, calcitonin, adrenomedulin, somatostatin, GHRH, CRF, ACTH, GRP, PTH, VIP (vasoactive intestinal and related polypeptide), somatostatin, dopamine, motilin, amylin, bradykinin, CGRP (calcitonin gene-related peptide), leukotrienes, pancreastatin, prostaglandins, thromboxane, adenosine, adrenaline, α and β-chemokines (e.g., IL-8, GROα, GROβ, GROγ, NAP-2, ENA-78, PF4, IP10, GCP-2, MCP-1, HC14, MCP-3, I-309, MIP-1α, MIP-1β, RANTES, etc.), endothelin, enterogastrin, histamine, neurotensin, TRH, pancreatic polypeptide, galanin, etc.) as well as other substances, for example, tissue extracts and cell culture supernatants from human and mammals (e.g., mice, rats, swine, bovine, sheep, monkeys, etc.). For example, the tissue extract or cell culture supernatant is added to the receptor protein of the present invention and fractionated while assaying the cell stimulating activities, etc. to finally give a single ligand.

In more detail, the method for determining ligands of the present invention comprises determining compounds (e.g., peptides, proteins, non-peptide compounds, synthetic compounds, fermentation products, etc.) or salts thereof that bind to the receptor protein of the present invention to provide cell stimulating activities (e.g., the activities that accelerate or suppress arachidonic acid release, acetylcholine release, intracellular $Ca^{2+}$ release, intracellular cAMP production, intracellular cGMP production, inositol phosphate production, change in cell membrane potential, phosphorylation of intracellular proteins, activation of c-fos, pH reduction, etc.), using the receptor of the present invention, its partial peptides or salts thereof, or by the receptor binding assay using the constructed recombinant receptor protein expression system.

The method for determining ligands of the present invention is characterized, for example, by measurement of the amount of the test compound bound to the receptor protein or the partial peptide, or by assaying the cell-stimulating activities, etc., when the test compound is brought in contact with the receptor protein of the present invention or its partial peptides.

More specifically, the present invention provides the following features:

(1) a method for determining a ligand to the receptor protein of the present invention or its salt, which comprises bringing a labeled test compound in contact with the receptor protein of the present invention or its salt or the partial peptide of the present invention or its salt and measuring the amount of the labeled test compound bound to the receptor protein or its salt or to the partial peptide or its salt;

(2) a method for determining ligands to the receptor protein of the present invention or its salt, which comprises bringing a labeled test compound in contact with cells or cell membrane fraction containing the receptor protein of the present invention, and measuring the amount of the labeled test compound bound to the cells or the membrane fraction;

(3) a method for determining ligands to the receptor protein of the present invention, which comprises culturing a transformant containing the DNA encoding the receptor protein of the present invention, bringing a labeled test compound in contact with the receptor protein expressed on the cell membrane by said culturing, and measuring the amount of the labeled test compound bound to the receptor protein or its salt;

(4) a method for determining ligands to the receptor protein of the present invention or its salt, which comprises bringing a test compound in contact with cells containing the receptor protein of the present invention and measuring the receptor protein-mediated cell stimulating activities (e.g., the activities that promote or suppress arachidonic acid release, acetylcholine release, intracellular $Ca^{2+}$ release, intracellular cAMP production, intracellular cGMP production, inositol phosphate production, change in cell membrane potential, phosphorylation of intracellular proteins, activation of c-fos, pH reduction, etc.); and,

(5) a method for determining ligands to the receptor protein of the present invention or its salt, which comprises culturing a transformant containing DNA encoding the receptor protein of the present invention, bringing a labeled test compound in contact with the receptor protein expressed on the cell membrane by said culturing, and measuring the receptor protein-mediated cell stimulating activities (e.g., the activities that promote or suppress arachidonic acid release, acetylcholine release, intracellular $Ca^{2+}$ release, intracellular cAMP production, intracellular cGMP production, inositol phosphate production, change in cell membrane potential, phosphorylation of intracellular proteins, activation of c-fos, pH reduction, etc.).

It is particularly preferred to perform the tests (1) to (3) described above, thereby to confirm that the test compound can bind to the receptor protein of the present invention, followed by the tests (4) and (5) described above.

Any protein exemplified to be usable as the receptor protein for determining ligands, so long as it contains the receptor protein of the present invention or the partial peptide of the present invention. However, the receptor protein that is abundantly expressed using animal cells is appropriate.

The receptor protein of the present invention can be manufactured by the method for expression described above, preferably by expressing DNA encoding the receptor protein in mammalian or insect cells. As DNA fragments encoding the desired portion of the protein, complementary DNA is generally used but not necessarily limited thereto. For example, gene fragments or synthetic DNA may also be used. For introducing a DNA fragment encoding the receptor protein of the present invention into host animal cells and efficiently expressing the same, it is preferred to insert the DNA fragment downstream a polyhedrin promoter of nuclear polyhedrosis virus (NPV), which is a baculovirus having insect hosts, an SV40-derived promoter, a retrovirus promoter, a metallothionein promoter, a human heat shock promoter, a cytomegalovirus promoter, an SR $\alpha$ promoter or the like. The amount and quality of the receptor expressed can be determined by a publicly known method. For example, this determination can be made by the method described in the literature (Nambi, P., et al., J. Biol. Chem., <u>267,</u> 19555-19559 (1992)).

Accordingly, the subject containing the receptor protein of the present invention, its partial peptides or salts thereof in the method for determining the ligand according to the present invention may be the receptor protein, its partial peptides or salts thereof purified by publicly known methods, cells containing the receptor protein, or membrane fractions of such cells.

Where cells containing the receptor protein of the present invention are used in the method of the present invention for determination of ligands, the cells may be fixed using glutaraldehyde, formalin, etc. The fixation can be made by a publicly known method.

The cells containing the receptor protein of the present invention are host cells that have expressed the receptor protein of the present invention, which host cells include Escherichia coli, Bacillus subtilis, yeast, insect cells, animal cells, and the like.

The cell membrane fraction refers to a fraction abundant in cell membrane obtained by cell disruption and subsequent fractionation by a publicly known method. Useful cell disruption methods include cell squashing using a Potter-Elvehjem homogenizer, disruption using a Waring blender or Polytron (manufactured by Kinematica Inc.), disruption by ultrasonication, and disruption by cell spraying through thin nozzles under an increased pressure using a French press or the like. Cell membrane fractionation is effected mainly by fractionation using a centrifugal force, such as centrifugation for fractionation and density gradient centrifugation. For example, cell disruption fluid is centrifuged at a low speed (500 rpm to 3,000 rpm) for a short period of time (normally about 1 to about 10 minutes), the resulting supernatant is then centrifuged at a higher speed (15,000 rpm to 30,000 rpm) normally for 30 minutes to 2 hours. The precipitate thus obtained is used as the membrane fraction. The membrane fraction is rich in the receptor protein expressed and membrane components such as cell-derived phospholipids and membrane proteins.

The amount of the receptor protein in the cells containing the receptor protein and in the membrane fraction is preferably $10^3$ to $10^8$ molecules per cell, more preferably $10^5$ to $10^7$ molecules per cell. As the amount of expression increases, the ligand binding activity per unit of membrane fraction (specific activity) increases so that not only the highly sensitive screening system can be constructed but also large quantities of samples can be assayed with the same lot.

To perform the methods (1) through (3) supra for determination of a ligand to the receptor protein of the present invention or its salt, an appropriate receptor fraction and a labeled test compound are required.

The receptor protein fraction is preferably a fraction of naturally occurring receptor protein or a recombinant receptor fraction having an activity equivalent to that of the natural protein. Herein, the term "equivalent activity" is intended to mean a ligand binding activity, a signal transduction activity or the like that is equivalent to that possessed by naturally occurring receptor proteins.

Preferred examples of labeled test compounds include angiotensin, bombesin, canavinoid, cholecystokinin, glutamine, serotonin, melatonin, neuropeptide Y, opioid, purines, vasopressin, oxytocin, PACAP, secretin, glucagon, calcitonin, adrenomedulin, somatostatin, GHRH, CRF, ACTH, GRP, PTH, VIP (vasoactive intestinal polypeptide), somatostatin, dopamine, motilin, amylin, bradykinin, CGRP (calcitonin gene-related peptide), leukotrienes, pancreastatin, prostaglandins, thromboxane, adenosine, adrenaline, $\alpha$ and $\beta$-chemokines (e.g., IL-8, GRO$\alpha$, GRO$\beta$, GRO$\gamma$, NAP-2, ENA-78, PF4, IP10, GCP-2, MCP-1, HC14, MCP-3, I-309, MIP1$\alpha$, MIP-1$\beta$, RANTES, etc.), endothelin, enterogastrin, histamin, neurotensin, TRH, pancreatic polypeptide, galanin, etc.), which are labeled with [$^3$H], [$^{125}$I], [$^{14}$C], [$^{35}$S], etc.

More specifically, the ligand to the receptor protein of the present invention or its salt is determined by the following procedures. First, a standard receptor preparation is prepared by suspending cells containing the receptor protein of the present invention or the membrane fraction thereof in a buffer appropriate for use in the determination method. Any buffer can be used so long as it does not inhibit the ligand-receptor binding, such buffers including a phosphate buffer or a Tris-HCl buffer having pH of 4 to 10 (preferably pH of 6 to 8). For the purpose of minimizing non-specific binding, a surfactant such as CHAPS, Tween - 80™ (manufactured by Kao-Atlas Inc.), digitonin or deoxycholate, and various proteins such as bovine serum albumin or gelatin, may optionally be added to the buffer. Further for the purpose of suppressing the degradation of the receptors or ligands by proteases, a protease inhibitor such as PMSF, leupeptin, E-64 (manufactured by Peptide Institute, Inc.) and pepstatin may also be added. A given amount (5,000 to 500,000 cpm) of the test compound labeled with [$^3$H], [$^{125}$I], [$^{14}$C], [$^{35}$S] or the like is added to 0.01 ml to 10 ml of the receptor solution. To determine the amount of non-specific binding (NSB), a reaction tube containing an unlabeled test compound in a large excess is also prepared. The reaction is carried out at approximately 0 to 50°C, preferably about 4 to 37°C for about 20 minutes to about 24 hours, preferably about 30 minutes to about 3 hours. After completion of the reaction, the reaction mixture is filtrated through glass fiber filter paper, etc. and washed with an appropriate volume of the same buffer. The residual radioactivity on the glass fiber filter paper is then measured by means of a liquid scintillation counter or $\gamma$-counter. A test compound exceeding 0 cpm in count obtained by subtracting non-specific binding (NSB) from the total binding (B) (B minus NSB) may be selected as a ligand (agonist) to the receptor protein of the present invention or its salt.

The method (4) or (5) above for determination of a ligand to the receptor protein of the present invention or its salt can be performed as follows. The receptor protein-mediated cell-stimulating activities (e.g., the activities that promote or suppress arachidonic acid release, acetylcholine release, intracellular Ca$^{2+}$ release, intracellular CAMP production, intracellular cGMP production, inositol phosphate production, change in cell membrane potential, phosphorylation of intracellular proteins, activation of c-fos, pH reduction, etc.) may be determined by a publicly known method, or using an assay kit commercially available. Specifically, cells containing the receptor protein are first cultured on a multi-well plate, etc. Prior to the ligand determination, the medium is replaced with fresh medium or with an appropriate non-cytotoxic buffer, followed by incubation for a given period of time in the presence of a test compound, etc. Subsequently, the cells are extracted or the supernatant is recovered and the resulting product is quantified by appropriate procedures. Where it is difficult to detect the production of the index substance (e.g., arachidonic acid) for the cell-stimulating activity due to a degrading enzyme contained in the cells, an inhibitor against such a degrading enzyme may be added prior to the assay. For detecting activities such as the cAMP production suppression activity, the baseline production in the cells is increased by forskolin or the like and the suppressing effect on the increased baseline production may then be detected.

The kit of the present invention for determination of the ligand that binds to the receptor protein of the present invention or its salt comprises the receptor protein of the present invention or its salt, the partial peptide of the present invention or its salt, cells containing the receptor protein of the present invention, or the membrane fraction of the cells containing the receptor protein of the present invention.

Examples of the ligand determination kit of the present invention are given below.

1. Reagents for determining ligands

(1) Buffers for assay and washing
Hanks' Balanced Salt Solution (manufactured by Gibco Co.) supplemented with 0.05% bovine serum albumin (Sigma Co.).
The solution is sterilized by filtration through a 0.45 $\mu$m filter and stored at 4°C. Alternatively, the solution may be prepared at use.

(2) Standard G protein-coupled receptor protein
CHO cells on which the receptor protein of the present invention has been expressed are passaged in a 12-well plate in a density of $5 \times 10^5$ cells/well followed by culturing at 37°C under 5% CO$_2$ and 95% air for 2 days.

(3) Labeled test compounds
Compounds labeled with [$^3$H], [$^{125}$I], [$^{14}$C], [$^{35}$S], etc., which are commercially available labels, or compounds labeled by appropriate methods.
An aqueous solution of the compound is stored at 4°C or -20°C. The solution is diluted to 1 $\mu$M with an assay buffer at use. A sparingly water-soluble test compound is dissolved in dimethylformamide, DMSO, methanol, etc.

(4) Non-labeled compounds
A non-labeled form of the same compound as the labeled compound is prepared in a concentration 100 to 1,000-fold higher than that of the labeled compound.

2. Method for assay

(1) CHO cells expressing the receptor protein of the present invention are cultured in a 12-well culture plate. After washing twice with 1 ml of an assay buffer, 490 µl of the assay buffer is added to each well.
(2) After 5 µl of the labeled test compound is added, the resulting mixture is incubated at room temperature for an hour. To determine the non-specific binding, 5 µl of the non-labeled compound is added to the system.
(3) The reaction mixture is removed and the wells are washed 3 times with 1 ml of washing buffer. The labeled test compound bound to the cells is dissolved in 0.2N NaOH-1% SDS and then mixed with 4 ml of liquid scintillator A (manufactured by Wako Pure Chemical Industries, Ltd.).
(4) The radioactivity is measured using a liquid scintillation counter (manufactured by Beckman Co.).

The ligands that bind to the receptor protein of the present invention or its salt include substances specifically present in the brain, pituitary gland and pancreas. Examples of such ligands are angiotensin, bombesin, canavinoid, cholecystokinin, glutamine, serotonin, melatonin, neuropeptide Y, opioids, purines, vasopressin, oxytocin, PACAP, secretin, glucagon, calcitonin, adrenomedulin, somatostatin, GHRH, CRF, ACTH, GRP, PTH, VIP (vasoactive intestinal peptide), somatostatin, dopamine, motilin, amylin, bradykinin, CGRP (calcitonin gene-related peptide), leukotriens, pancreastatin, prostaglandins, thromboxane, adenosine, adrenaline, $\alpha$ and $\beta$-chemokines (e.g., IL-8, GRO$\alpha$, GRO$\beta$, GRO$\gamma$, NAP-2, ENA-78, PF4, IP10, GCP-2, MCP-1, HC14, MCP-3, I-309, MIP1$\alpha$, MIP-1$\beta$, RANTES, etc.), endothelin, enterogastrin, histamine, neurotensin, TRH, pancreatic polypeptide, galanin, etc.

(2) Prophylactic and/or therapeutic agents for diseases associated with dysfunction of the G protein-coupled receptor protein of the present invention

When a compound is clarified to be a ligand of the receptor protein of the present invention by the methods described in (1), ① the receptor protein *of* the present invention, or ② the DNA encoding the receptor protein can be used, depending on the activities possessed by the ligand, as a prophylactic and/or therapeutic agent for diseases associated with dysfunction of the receptor protein of the present invention.
For example, when the physiological activity of the ligand cannot be expected in a patient (deficiency of the receptor protein) due to a decrease in the receptor protein of the present invention, the activity of the ligand can be exhibited by: ① administering the receptor protein of the present invention to the patient thereby to supplement the amount of the receptor protein; or ② by increasing the amount of the receptor protein in the patient through: i) administration of the DNA encoding the receptor protein of the present invention to express the same in the patient; or ii) insertion and expression of the DNA encoding the receptor protein of the present invention in the objective cells to transplant the cells to the patient, whereby the activity of the ligand can be sufficiently exhibited. That is, the DNA encoding the receptor protein of the present invention is useful as a safe and low toxic prophylactic and/or therapeutic agent for diseases associated with dysfunction of the receptor protein of the present invention.
The receptor protein of the present invention is recognized to have about 60% homology to SLC-receptor, which is a G protein-coupled receptor protein, or somatostatin receptor type 3 or type 5 (SS5R or SS3R), on an amino acid sequence level.
The receptor protein of the present invention and the DNA encoding the receptor protein of the present invention are useful for the prevention and/or treatment of central dysfunction (e.g., Alzheimer's disease, senile dementia, suppression of eating (anorexia), epilepsy, etc.), hormone diseases (e.g., weak pains, atonic bleeding, before and after expulsion, subinvolution of uterus, cesarean section, induced abortion, galactostasis, etc.), liver/gallbladder/pancreas/endocrine-associated diseases (e.g., diabetes mellitus, suppression of eating, etc.), inflammatory diseases (e.g., allergy, asthma, rheumatoid, etc.), circulatory diseases (e.g., hypertension, cardiac hypertrophy, angina pectoris, arteriosclerosis, etc.).
When the receptor protein of the present invention is used as the prophylactic/therapeutic agents supra, the receptor protein can be prepared into a pharmaceutical composition in a conventional manner.
On the other hand, where the DNA encoding the receptor protein of the present invention (hereinafter sometimes referred to as the DNA of the present invention) is used as the prophylactic/therapeutic agents described above, the DNA itself is administered; alternatively, the DNA is inserted into an appropriate vector such as retrovirus vector, adenovirus vector, adenovirus-associated virus vector, etc. and then administered in a conventional manner. The DNA of the present invention may also be administered as naked DNA, or with adjuvants to assist its uptake by gene gun or through a catheter such as a catheter with a hydrogel.
For example, ① the receptor protein of the present invention or ② the DNA encoding the receptor protein can be used orally, for example, in the form of tablets which may be sugar coated if necessary and desired, capsules, elixirs, microcapsules etc., or parenterally in the form of injectable preparations such as a sterile solution and a suspension in water or with other pharmaceutically acceptable liquid. These preparations can be manufactured by mixing ① the receptor protein of

the present invention or ② the DNA encoding the receptor protein with a physiologically acceptable known carrier, a flavoring agent, an excipient, a vehicle, an antiseptic agent, a stabilizer, a binder, etc. in a unit dosage form required in a generally accepted manner that is applied to making pharmaceutical preparations. The effective component in the preparation is controlled in such a dose that an appropriate dose is obtained within the specified range given.

Additives miscible with tablets, capsules, etc. include a binder such as gelatin, corn starch, tragacanth and gum arabic, an excipient such as crystalline cellulose, a swelling agent such as corn starch, gelatin and alginic acid, a lubricant such as magnesium stearate, a sweetening agent such as sucrose, lactose and saccharin, and a flavoring agent such as peppermint, akamono oil and cherry. When the unit dosage is in the form of capsules, liquid carriers such as oils and fats may further be used together with the additives described above. A sterile composition for injection may be formulated by conventional procedures used to make pharmaceutical compositions, e.g., by dissolving or suspending the active ingredients in a vehicle such as water for injection with a naturally occurring vegetable oil such as sesame oil and coconut oil, etc. to prepare the pharmaceutical composition. Examples of an aqueous medium for injection include physiological saline and an isotonic solution containing glucose and other auxiliary agents (e. g., D-sorbitol, D-mannitol, sodium chloride, etc.) and may be used in combination with an appropriate dissolution aid such as an alcohol (e.g., ethanol or the like), a polyalcohol (e.g., propylene glycol and polyethylene glycol), a nonionic surfactant (e.g., polysorbate 80™ and HCO-50), etc. Examples of the oily medium include sesame oil and soybean oil, which may also be used in combination with a dissolution aid such as benzyl benzoate and benzyl alcohol.

The prophylactic/therapeutic agent described above may further be formulated with a buffer (e.g., phosphate buffer, sodium acetate buffer, etc.), a soothing agent (e.g., benzalkonium chloride, procaine hydrochloride, etc.), a stabilizer (e.g., human serum albumin, polyethylene glycol, etc.), a preservative (e.g., benzyl alcohol, phenol, etc.), an antioxidant, etc. The thus-prepared liquid for injection is normally filled in an appropriate ampoule.

Since the thus obtained pharmaceutical preparation is safe and low toxic, the preparation can be administered to human or mammal (e.g., rats, rabbits, sheep, swine, bovine, cats, dogs, monkeys, etc.).

The dose of the receptor protein of the present invention varies depending on subject to be administered, organs to be administered, conditions, routes for administration, etc.; in oral administration, e.g., for the patient with suppression of eating, the dose is normally about 0.1 mg to about 100 mg, preferably about 1.0 to about 50 mg, and more preferably about 1.0 to about 20 mg per day (as 60 kg body weight). In parenteral administration, the single dose varies depending on subject to be administered, target organ, conditions, routes for administration, etc. but it is advantageous, e.g., for the patient with suppression of eating, to administer the active ingredient intravenously in a daily dose of about 0.01 to about 30 mg, preferably about 0.1 to about 20 mg, and more preferably about 0.1 to about 10 mg (as 60 kg body weight). For other animal species, the corresponding dose as converted per 60 kg body weight can be administered.

The dose of the DNA of the present invention varies depending on subject to be administered, organs to be administered, conditions, routes for administration, etc.; in oral administration, e.g., for the patient with suppression of eating, the dose is normally about 0.1 mg to about 100 mg, preferably about 1.0 to about 50 mg, and more preferably about 1.0 to about 20 mg per day (as 60 kg body weight). In parenteral administration, the single dose varies depending on subject to be administered, target organ, conditions, routes for administration, etc. but it is advantageous, e.g., for the patient with suppression of eating, to administer the active ingredient intravenously in a daily dose of about 0.01 to about 30 mg, preferably about 0.1 to about 20 mg, and more preferably about 0.1 to about 10 mg (as 60 kg body weight). For other animal species, the corresponding dose as converted per 60 kg body weight can be administered.

(3) Gene diagnostic agent

By using the DNA of the present invention as a probe, an abnormality (gene abnormality) of the DNA or mRNA encoding the receptor protein of the present invention or its partial peptide in human or mammal (e. g., rats, rabbits, sheep, swine, bovine, cats, dogs, monkeys, etc.) can be detected. Therefore, the DNA of the present invention is useful as a gene diagnostic agent for the damage against the DNA or mRNA, its mutation, or its decreased expression, or increased expression or overexpression of the DNA or mRNA.

The gene diagnosis described above using the DNA of the present invention can be performed by, for example, the publicly known Northern hybridization assay or the PCR-SSCP assay (Genomics, 5, 874-879 (1989); Proceedings of the National Academy of Sciences of the United States of America, 86, 2766-2770 (1989)).

(4) Methods of screening compounds that alter the expression level of the receptor protein of the present invention or its partial peptide

By using the DNA of the present invention as a probe, the DNA can be used for screening of compounds that alter the amount of the receptor protein of the present invention or its partial peptide .

That is, the present invention provides methods of screening compounds that alter the amount of the receptor protein or its partial peptide, which comprises measuring the amount of mRNA in the receptor protein of the present invention or its partial peptide contained in, for example, (i) ① blood, ② specific organs, ③ tissues or cells isolated from the organs of non-human mammals, or in (ii) transformants, etc.

The amount of mRNA in the receptor protein of the present invention or its partial peptide can be specifically measured as follows.

(i) Normal or disease models of non-human mammals (e.g., mice, rats, rabbits, sheep, swine, bovine, cats, dogs, monkeys, more specifically, rats with dementia, obese mice, rabbits with arteriosclerosis, tumor-bearing mice, etc.) receive administration of a drug (e.g., anti-dementia agents, hypotensive agents, anticancer agents, antiobestic agents, etc.) or physical stress (e.g., soaking stress, electric shock, light and darkness, low temperature, etc.), and the blood, specific organs (e.g., brain, liver, kidneys, etc.), or tissues or cells isolated from the organs are obtained after a specified period of time.

The mRNA of the receptor protein of the present invention or its partial peptide contained in the thus obtained cells is extracted from the cells, for example, in a conventional manner and quantified using, e.g., TaqManPCR, or may also be analyzed by northern blot technique by publicly known methods.

(ii) Transformants that express the receptor protein of the present invention or its partial peptide are prepared according to the methods described above, and the mRNA of the receptor protein of the present invention or its partial peptide can be quantified and analyzed, as described above.

Compounds that alter the expression level of the receptor protein of the present invention or its partial peptide can be screened by the following procedures.

(i) To normal or disease models of non-human mammals, a test compound is administered at a specified period of time before (30 minutes to 24 hours before, preferably 30 minutes to 12 hours before, more preferably 1 hour to 6 hours before), at a specified time after (30 minutes to 3 days after, preferably 1 hour to 2 days after, more preferably 1 hour to 24 hours after), or simultaneously with a drug or physical stress. At a specified time (30 minute to 3 days, preferably 1 hour to 2 days, more preferably 1 hour to 24 hours) after administration of the test compound, the amount of mRNA in the receptor protein of the present invention or its partial peptide contained in cells are quantified and analyzed.

(ii) Transformants are cultured in a conventional manner and a test compound is mixed in the culture medium. After a specified time (after 1 day to 7 days, preferably after 1 day to 3 days, more preferably after 2 to 3 days), the amount of mRNA in the receptor protein of the present invention or its partial peptide contained in the transformants can be quantified and analyzed.

The compounds or their salts, which are obtainable by the screening methods of the present invention, are compounds that alter the expression level of the receptor protein of the present invention or its partial peptide . Specifically, (a) compounds that potentiate the cell stimulating activities mediated by the G protein-coupled receptor (e.g., activities that promote or suppress arachidonic acid release, acetylcholine release, intracellular $Ca^{2+}$ release, intracellular cAMP production, intracellular cGMP production, inositol phosphate production, alters in cell membrane potential, phosphorylation of intracellular proteins, activation of c-fos, pH reduction, etc.) by increasing the expression level of the receptor protein of the present invention or its partial peptide ; and (b) compounds that decrease the cell-stimulating activities by reducing the expression level of the receptor protein of the present invention or its partial peptide .

The compounds include peptides, proteins, non-peptide compounds, synthetic compounds, and fermentation products. They may be novel or known compounds.

The compounds that increase the cell-stimulating activities are useful as safe and low-toxic pharmaceuticals for potentiation of the physiological activity of the receptor protein of the present invention (e.g., central dysfunction (e.g., Alzheimer's disease, senile dementia, suppression of eating (anorexia), epilepsy, etc.), hormone diseases (e.g., weak pains, atonic bleeding, before and after expulsion, subinvolution of uterus, cesarean section, induced abortion, galactostasis, etc.), liver/gallbladder/pancreas/endocrine-associated diseases (e.g., diabetes mellitus, suppression of eating, etc.), inflammatory diseases (e.g., allergy, asthma, rheumatoid, etc.), circulatory diseases (e.g., hypertension, cardiac hypertrophy, angina pectoris, arteriosclerosis, etc.).

The compounds that decrease the cell-stimulating activities are useful as safe and low-toxic pharmaceuticals for reducing the physiological activity of the receptor protein or its other forms of the present invention.

When the compounds or their salt forms, which are obtainable by the screening methods of the present invention, are used as pharmaceutical components, the compounds can be formulated by the conventional methods. For example, as described for the pharmaceuticals containing the receptor protein of the present invention, the compounds can be prepared into tablets, capsules, elixir, microcapsules, aseptic solution, or suspension.

The preparations obtained as described above are safe and low-toxic, and can be administered to human and mammals (e.g., rats, rabbits, sheep, swine, bovine, cats, dogs, monkeys, etc.).

The dose of the compounds or their salt forms varies depending on subject to be administered, target organs, conditions, routes for administration, etc.; in oral administration, e.g., for the patient with suppression of eating, the dose is normally about 0.1 mg to about 100 mg, preferably about 1.0 to about 50 mg, and more preferably about 1.0 to about 20 mg per day (as 60 kg body weight). In parenteral administration, the single dose varies depending on subject to be administered, target organ, conditions, routes for administration, etc. but it is advantageous, e.g., for the patient with suppression of eating, to administer the active ingredient intravenously in a daily dose of about 0.01 to about 30 mg, preferably about 0.1 to about 20 mg, and more preferably about 0.1 to about 10 mg (as 60 kg body weight). For other animal species, the corresponding dose as converted per 60 kg body weight can be administered.

(5) Prophylactic and/or therapeutic agents for various diseases comprising the compounds that alter the expression level of the receptor protein of the present invention or its partial peptide

As described above, the receptor protein of the present invention is considered to play some important role such as a role in the central function. Therefore, the compounds that alter the expression level of the receptor protein of the present invention or its partial peptide can be used as prophylactic and/or therapeutic agents for diseases associated with dysfunction of the receptor protein of the present invention (e.g., central dysfunction (e.g., Alzheimer's disease, senile dementia, suppression of eating (anorexia), epilepsy, etc.), hormone diseases (e.g., weak pains, atonic bleeding, before and after expulsion, subinvolution of uterus, cesarean section, induced abortion, galactostasis, etc.), liver/gallbladder/pancreas/endocrine-associated diseases (e.g., diabetes mellitus, suppression of eating, etc.), inflammatory diseases (e.g., allergy, asthma, rheumatoid, etc.), circulatory diseases (e.g., hypertension, cardiac hypertrophy, angina pectoris, arteriosclerosis, etc.).

Where these compounds are used as prophylactic and/or therapeutic agents for diseases associated with dysfunction of the receptor protein of the present invention, the preparations can be obtained by the conventional methods.

For example, the compounds can be administered orally as a sugar coated tablet, capsule, elixir, and microcapsule, or non-orally as injection such as aseptic solution or suspension in water or other pharmaceutically acceptable liquid. For example, preparations of the compounds can be manufactured by mixing with physiologically acceptable known carrier, flavor, filler, vehicle, antiseptic, stabilizer, and binder in a unit-dosage form required for generally approved drug preparation.

The amount of the active ingredient is set to an appropriate volume within the specified range.

For the additive that may be mixed in tablets and capsules, for example, binders such as gelatin, cornstarch, tragacanth, and acacia, fillers such as crystalline cellulose, imbibers such as cornstarch, gelatin, and alginic acid, lubricants such as magnesium stearate, sweeteners such as sucrose and saccharin, and flavors such as peppermint, akamono oil and cherry are used. When the dosage form is a capsule, liquid carrier such as fat and oil may be contained. Aseptic compositions for injection can be formulated following the usual preparation procedure such as dissolving or suspending the active substance in vehicle, e.g., water for injection, and natural plant oils e.g., sesame oil and coconut oil. For the aqueous solution for injection, for example, physiological saline and isotonic solutions (e.g., D-sorbitol, D-mannitol, sodium hydrochloride) containing glucose and other adjuvant are used. Appropriate dissolution-assisting agents, for example, alcohol (e.g., ethanol), polyalcohol (e.g., propylene glycol, polyethylene glycol), nonionic surfactant (e.g., polysorbate 80™, HCO-50) may be combined. For the oily solution, for example, sesame oil and soybean oil are used, and dissolution-assisting agents such as benzyl benzoate and benzyl alcohol may be combined.

The prophylactic/therapeutic agents described above may be combined with buffers (e.g., phosphate buffer, sodium acetate buffer), soothing agents (e.g., benzalkonium chloride, procaine hydrochloride), stabilizers (e.g., human serum albumin, polyethylene glycol), preservatives (e.g., benzyl alcohol, phenol), antioxidants, and the like. The preparation for injection is usually filled in appropriate ampoules.

The preparations obtained as described above are safe and low-toxic, and can be administered to, for example, humans and mammals (e.g., rats, rabbits, sheep, swine, bovine, cats, dogs, monkeys, etc.).

The dose of the compounds or their salt forms varies depending on subject to be administered, target organs, conditions, routes for administration, etc.; in oral administration, e.g., for the patient with suppression of eating, the dose is normally about 0.1 mg to about 100 mg, preferably about 1.0 to about 50 mg, and more preferably about 1.0 to about 20 mg per day (as 60 kg body weight). In parenteral administration, the single dose varies depending on subject to be administered, target organ, conditions, routes for administration, etc. but it is advantageous, e.g., for the patient with suppression of eating, to administer the active ingredient intravenously in a daily dose of about 0.01 to about 30 mg, preferably about 0.1 to about 20 mg, and more preferably about 0.1 to about 10 mg (as 60 kg body weight). For other animal species, the corresponding dose as converted per 60 kg body weight can be administered.

(6) Methods of quantifying ligands for the G protein-coupled protein of the present invention

Since the receptor protein of the present invention has binding affinity to ligands, the ligand concentration can be quantified in vivo with good sensitivity.

The quantification methods of the present invention can be used in combination with, for example, a competitive method. The ligand concentration in a test sample can be measured by contacting the test sample to the receptor protein of the present invention. Specifically, the methods can be used by following, for example, the methods described in ① and ② below or its modified methods.

① Hiroshi Irie, ed. "Radioimmunoassay," Kodansha, published in 1974
② Hiroshi Irie, ed. "Sequel to the Radioimmunoassay," Kodansha, published in 1979

(7) Methods of screening compounds (agonists, antagonists, or the like) that alter the binding property between the G protein-coupled receptor protein of the present invention and ligands

Using the receptor protein of the present invention, or using the receptor binding assay system of the expression system constructed using the recombinant receptor protein, compounds (e.g., peptides, proteins, non-peptide compounds, synthetic compounds, fermentation products, etc.) or salt forms thereof that alter the binding property between ligands and the receptor protein of the present invention can be efficiently screened.

Such compounds include (a) compounds that have the G protein-coupled receptor-mediated cell-stimulating activities (e.g., activities that promote or suppress arachidonic acid release, acetylcholine release, intracellular $Ca^{2+}$ release, intracellular CAMP production, intracellular cGMP production, inositol phosphate production, changes in cell membrane potential, phosphorylation of intracellular proteins, activation of c-fos, pH reduction, etc.) (so-called agonists to the receptor protein of the present invention); (b) compounds that do not have the cell-stimulating activity (so-called antagonists to the receptor protein of the present invention); (c) compounds that potentiate the binding affinity between ligands and the G protein-coupled receptor protein of the present invention; and (d) compounds that reduce the binding affinity between ligands and the G protein-coupled receptor protein of the present invention (it is preferred to screen the compounds described in (a) using the ligand determination methods described above).

That is, the present invention provides methods of screening compounds or their salt forms that alter the binding property between ligands and the receptor protein, its partial peptide or salts thereof, which comprises comparing (i) the case wherein the receptor protein of the present invention, its partial peptide or salts thereof are brought in contact with a ligand, with (ii) the case wherein the receptor protein of the present invention, its partial peptide or salts thereof are brought in contact with a ligand and a test compound.

The screening methods of the present invention are characterized by assaying, for example, the amount of ligand bound to the receptor protein, the cell-stimulating activity, etc., and comparing the property between (i) and (ii).

More specifically, the present invention provides the following screening methods:

① a method of screening a compound or its salt that alters the binding property between a ligand and the receptor protein of the present invention, which comprises:

measuring the amount of a labeled ligand bound to the receptor protein, when the labeled ligand is brought in contact with the receptor protein of the present invention and when the labeled ligand and a test compound are brought in contact with the receptor protein of the present invention, and, comparing the binding property between them;

② a method of screening a compound or its salt that alters the binding property between a ligand and the receptor protein of the present invention, which comprises:

measuring the amount of a labeled ligand bound to cells or the membrane fraction of the cells, when the labeled ligand is brought in contact with the cells or cell membrane fraction containing the receptor protein of the present invention and when the labeled ligand and a test compound are brought in contact with the cells or cell membrane fraction containing the receptor protein of the present invention, and, comparing the binding property between them;

③ a method of screening a compound or its salt that alters the binding property between a ligand and the receptor protein of the present invention, which comprises:

measuring the amount of a labeled ligand to the receptor protein, when the labeled ligand is brought in contact with the receptor protein expressed on the cell membrane induced by culturing a transformant containing the DNA of the present invention and when the labeled ligand and a test compound are brought in contact with the receptor protein of the present invention expressed on the cell membrane induced by culturing a transformant containing the DNA of the present invention, and,

comparing the binding property between them;

④ a method of screening a compound or its salt that alters the binding property between a ligand and the receptor protein of the present invention, which comprises:

measuring the receptor-mediated cell-stimulating activity (e.g., the activity that promotes or suppresses arachidonic acid release, acetylcholine release, intracellular $Ca^{2+}$ release, intracellular CAMP production, intracellular cGMP production, inositol phosphate production, changes in cell membrane potential, phosphorylation of intracellular proteins, activation of c-fos, pH reduction, etc.), when a compound (e.g., a ligand to the receptor protein of the present invention) that activates the receptor protein of the present invention is brought in contact with cells containing the receptor protein of the present invention and when the compound that activates the receptor protein of the present invention and a test compound are brought in contact with cells containing the receptor protein of the present invention, and, comparing the binding property between them; and,

⑤ a method of screening a compound or its salt that alters the binding property between a ligand and the receptor protein of the present invention, which comprises:

measuring the receptor-mediated cell-stimulating activity (e.g., the activity that promotes or suppresses arachidonic acid release, acetylcholine release, intracellular $Ca^{2+}$ release, intracellular CAMP production, intracellular cGMP production, inositol phosphate production, changes in cell membrane potential, phosphorylation of intracellular proteins, activation of c-fos, pH reduction, etc.), when a compound (e.g., a ligand for the receptor protein of the present invention) that activates the receptor protein of the present invention is brought in contact with the receptor protein of the present invention expressed on the cell membrane induced by culturing a transformant containing the DNA of the present invention and when the compound that activates the receptor protein of the present invention and a test compound are brought in contact with the receptor protein of the present invention expressed on the cell membrane induced by culturing a transformant containing the DNA of the present invention, and,

comparing the binding property between them.

Before the receptor protein of the present invention was obtained, it was required for screening G protein-coupled receptor agonists or antagonists to obtain candidate compounds first, using cells or tissues containing the G protein-coupled receptor protein or the cell membrane fraction from rats or other animals (primary screening), and then examine the candidate compounds whether the compounds actually inhibit the binding between human G protein-coupled receptor protein and ligands (secondary screening). When cells, tissues, or the cell membrane fractions were directly used, it was practically difficult to screen agonists or antagonists to the objective receptor protein, since other receptor proteins were present together.

However, using, for example, the human-derived receptor protein of the present invention, the primary screening becomes unnecessary, and compounds that inhibit the binding between ligands and the G protein-coupled receptor protein can be efficiently screened. Furthermore, it is easy to assess whether the obtained compound is an agonist or antagonist.

Hereinafter, the screening methods of the present invention are described more specifically.

First, for the receptor protein of the present invention used for the screening methods of the present invention, any substance may be used so long as it contains the receptor protein of the present invention described above. The cell membrane fraction from mammalian organs containing the receptor protein of the present invention is preferred. However, it is very difficult to obtain human organs. It is thus preferable to use rat-derived receptor proteins or the like, produced by large-scale expression using recombinants.

To manufacture the receptor protein of the present invention, the methods described above are used, and it is preferred to express the DNA of the present invention in mammalian and insect cells. For the DNA fragment encoding the objective protein region, the complementary DNA, but not necessarily limited thereto, is employed. For example, the gene fragments and synthetic DNA may also be used. To introduce a DNA fragment encoding the receptor protein of the present invention into host animal cells and efficiently express the DNA there, it is preferred to insert the DNA fragment downstream of a polyhedorin promoter of nuclear polyhedrosis virus (NPV) belonging to baculovirus hosted by insects, SV40-derived promoter, retrovirus promoter, metallothionein promoter, human heat shock promoter, cytomegalovirus promoter, or SRα promoter. The amount and quality of the expressed receptor are examined by publicly known methods, for example, the method described in the literature [Nambi, P. et al., The Journal of Biological Chemistry (J. Biol. Chem.), 267, 19555-19559, 1992].

Therefore, in the screening methods of the present invention, the material that contains the receptor protein of the present invention may be the receptor protein purified by publicly known methods, cells containing the receptor protein, or the cell membrane fraction containing the receptor protein.

In the screening methods of the present invention, when cells containing the receptor protein of the present invention are used, the cells may be fixed with glutaraldehyde, formalin, etc. The cells can be fixed by publicly known methods.

The cells containing the receptor protein of the present invention are host cells that express the receptor protein. For the host cells, Escherichia coli, Bacillus subtilis, yeast, insect cells, animal cells and the like are preferred.

The cell membrane fraction refers to a fraction abundant in cell membrane obtained by cell disruption and subsequent fractionation by a publicly known method. Useful cell disruption methods include cell squashing using a Potter-Elvehjem homogenizer, disruption using a Waring blender or Polytron (manufactured by Kinematica Inc.), disruption by ultrasonication, and disruption by cell spraying through thin nozzles under an increased pressure using a French press or the like. Cell membrane fractionation is effected mainly by fractionation using a centrifugal force, such as centrifugation for fractionation and density gradient centrifugation. For example, cell disruption fluid is centrifuged at a low speed (500 rpm to 3,000 rpm) for a short period of time (normally about 1 to about 10 minutes), the resulting supernatant is then centrifuged at a higher speed (15,000 rpm to 30,000 rpm) normally for 30 minutes to 2 hours. The precipitate thus obtained is used as the membrane fraction. The membrane fraction is rich in the receptor protein expressed and membrane components such as cell-derived phospholipids and membrane proteins.

The amount of the receptor protein in the cells containing the receptor protein and in the membrane fraction is preferably $10^3$ to $10^8$ molecules per cell, more preferably $10^5$ to $10^7$ molecules per cell. As the amount of expression increases, the ligand binding activity per unit of membrane fraction (specific activity) increases so that not only the highly sensitive screening system can be constructed but also large quantities of samples can be assayed with the same lot.

To screen the compounds that alter the binding property between ligands and the receptor protein of the present invention described in ① to ③ , for example, an appropriate receptor protein fraction and a labeled ligand are necessary.

The receptor protein fraction is preferably a fraction of naturally occurring receptor protein or a recombinant receptor fraction having an activity equivalent to that of the natural protein. Herein, the equivalent activity is intended to mean a ligand binding activity, a signal transduction activity or the like that is equivalent to that possessed by naturally occurring receptor proteins.

For the labeled ligand, a labeled ligand and a labeled ligand analogue are used. For example, ligands labeled with [$^3$H], [$^{125}$I], [$^{14}$C], [$^{35}$S], etc. are used.

Specifically, to screen the compounds that alter the binding property between ligands and the receptor protein of the present invention, first, the receptor protein standard is prepared by suspending cells or cell membrane fraction containing the receptor protein of the present invention in a buffer appropriate for the screening. For the buffer, any buffer that does not interfere with the binding of ligands to the receptor protein is usable and examples of such a buffer are phosphate buffer, Tris-hydrochloride buffer, etc., having pH of 4 to 10 (preferably pH of 6 to 8). To minimize a non-specific binding, a surfactant such as CHAPS, Tween - 80™ (Kao-Atlas Co.), digitonin, deoxycholate, etc. may be added to the buffer. To inhibit degradation of the receptor and ligands by proteases, protease inhibitors such as PMSF, leupeptin, E-64 (manufactured by Peptide Research Laboratory, Co.), and pepstatin may be added. To 0.01 to 10 ml of the receptor solution, a given amount (5,000 to 500,000 cpm) of labeled ligand is added, and $10^{-4}$ M - $10^{-10}$ M of a test compound is simultaneously added to be copresent. To examine non-specific binding (NSB), a reaction tube containing an unlabeled test compound in a large excess is also prepared. The reaction is carried out at approximately 0 to 50°C, preferably about 4 to 37°C for about 20 minutes to about 24 hours, preferably about 30 minutes to about 3 hours. After completion of the reaction, the reaction mixture is filtrated through glass fiber filter paper, etc. and washed with an appropriate volume of the same buffer. The residual radioactivity on the glass fiber filter paper is then measured by means of a liquid scintillation counter or $\gamma$-counter. Regarding the count obtained by subtracting the amount of non-specific binding (NSB) from the count obtained in the absence of any competitive substance ($B_0$) as 100%, when the amount of specific binding (B-NSB) is, for example, 50% or less, the test compound can be selected as a candidate substance having a potential of competitive inhibition.

To perform the methods ④ and ⑤ supra of screening the compounds that alter the binding property between ligands and the receptor protein of the present invention, the receptor protein-mediated cell-stimulating activity (e.g., activity that promotes or inhibits arachidonic acid release, acetylcholine release, intracellular $Ca^{2+}$ release, intracellular cAMP production, intracellular cGMP production, inositol phosphate production, changes in cell membrane potential, phosphorylation of intracellular proteins, activation of c-fos, pH reduction, etc.) can be measured using publicly known methods or commercially available kits.

Specifically, the cells containing the receptor protein of the present invention are first cultured on a multi-well plate, etc. Prior to screening, the medium is replaced with fresh medium or with an appropriate non-cytotoxic buffer, followed by incubation for a given period of time in the presence of a test compound, etc. Subsequently, the cells are extracted or the supernatant is recovered and the resulting product is quantified by appropriate procedures. Where it is difficult to detect the production of the index substance (e.g., arachidonic acid) for the cell-stimulating activity due to a degrading enzyme contained in the cells, an inhibitor against such a degrading enzyme may be added prior to the assay. For detecting activities such as the CAMP production suppression activity, the baseline production in the cells is increased by forskolin or the like and the suppressing effect on the increased baseline production may then be detected.

Screening by assaying the cell-stimulating activity requires cells that have expressed an appropriate receptor protein. For the cells that have expressed the receptor protein of the present invention, the cell line possessing the native receptor protein of the present invention, the cell line expressing the recombinant receptor protein described above and the like are desirable.

For the test compound, for example, peptides, proteins, non-peptide compounds, synthetic compounds, fermentation products, cell extracts, plant extracts, and animal tissue extracts are used. These compounds may be novel or known compounds.

The kits for screening the compounds or their salts that alter the binding property between ligands and the receptor protein of the present invention comprise the receptor protein of the present invention, cells containing the receptor protein of the present invention, or the membrane fraction of cells containing the receptor protein of the present invention.

Examples of the screening kits of the present invention are as follow.

1. Reagents for screening

① Buffer for measurement and washing
Hanks' balanced salt solution (manufactured by Gibco Co.) supplemented with 0.05% bovine serum albumin (manufactured by Sigma Co.).
The solution is sterilized by filtration through a 0.45 $\mu$m filter, and stored at 4°C or may be prepared at use.

② Standard G protein-coupled receptor
CHO cells expressing the receptor protein of the present invention are passaged in a 12-well plate at a density of $5 \times 10^5$ cells/well followed by culturing at 37°C under 5% $CO_2$ and 95% air for 2 days.

③ Labeled ligands
Aqueous solutions of ligands labeled with commercially available [3H], [125I], [14C], [35S], etc. are stored at 4°C or -20°C, and diluted to 1 $\mu$M with the measurement buffer.

④ Standard ligand solution
The ligand is dissolved in and adjusted to 1 mM with PBS containing 0.1% bovine serum albumin (manufactured by Sigma Co.) and stored at -20°C.

2. Measurement method

① CHO cells expressing the receptor protein of the present invention are cultured in a 12-well culture plate and washed twice with 1 ml of the measurement buffer, and 490 $\mu$l of the measurement buffer is added to each well.

② After adding 5 $\mu$l of $10^{-3}$ - $10^{-10}$ M test compound solution, 5 $\mu$l of a labeled ligand is added to the mixture, and the cells are incubated at room temperature for an hour. To determine the amount of the non-specific binding, 5 $\mu$l of the non-labeled ligand is added in place of the test compound.

③ The reaction solution is removed, and the wells are washed 3 times with the washing buffer. The labeled ligand bound to the cells is dissolved in 0.2N NaOH-1% SDS, and mixed with 4 ml of liquid scintillator A (manufactured by Wako Pure Chemical Industries, Ltd.)

④ The radioactivity is measured using a liquid scintillation counter (manufactured by Beckman Co.), and the percent maximum binding (PMB) is calculated by the equation below.

$$PMB = [(B - NSB)/(B_0 - NSB)] \times 100$$

PMB:    Percent maximum binding
B:        Value obtained in the presence of a test compound
NSB :    Non-specific binding
$B_0$ :       Maximum binding

The compounds or their salts, which are obtainable using the screening methods or the screening kits of the present invention, are the compounds that alter the binding property between ligands and the receptor protein of the present invention. Specifically, these compounds are: (a) compounds that have the G protein-coupled receptor-mediated cell-stimulating activity (e. g., activity that promotes or inhibits arachidonic acid release, acetylcholine release, intracellular $Ca^{2+}$ release, intracellular CAMP production, intracellular cGMP production, inositol phosphate production, changes in cell membrane potential, phosphorylation of intracellular proteins, activation of c-fos, pH reduction, etc.) (so-called agonists to the receptor protein of the present invention); (b) compounds having no cell stimulating-activity (so-called antagonists to the receptor protein of the present invention); (c) compounds that increase the binding affinity between ligands and the G protein-coupled receptor protein of the present invention; and (d) compounds that reduce the binding affinity between ligands and the G protein-coupled receptor protein of the present invention.

The compounds may be peptides, proteins, non-peptide compounds, synthetic compounds, fermentation products, and may be novel or known compounds.

Since agonists to the receptor protein of the present invention have the same physiological activities as those of the ligands for the receptor protein of the present invention, the agonists are useful as safe and low-toxic pharmaceuticals, correspondingly to the ligand activities.

Since antagonists to the receptor protein of the present invention can suppress the physiological activities of ligands for the receptor protein of the present invention, the antagonists are useful as safe and low-toxic pharmaceuticals that inhibit the ligand activities.

The compounds that increase the binding affinity between ligands and the G protein-coupled receptor protein of the present invention are useful as safe and low-toxic pharmaceuticals to potentiate the physiological activities that the receptor protein of the present invention-possess for the ligands (prophylactic and/or therapeutic agents for, e.g., central dysfunction (e.g., Alzheimer's disease, senile dementia, suppression of eating (anorexia), epilepsy, etc.), hormone diseases (e. g., weak pains, atonic bleeding, before and after expulsion, subinvolution of uterus, cesarean section, induced abortion, galactostasis, etc.), liver/gallbladder/pancreas/endocrine-associated diseases (e.g., diabetes mellitus, suppression of eating, etc.), inflammatory diseases (e.g., allergy, asthma, rheumatoid, etc.), circulatory diseases (e.g., hypertension, cardiac hypertrophy, angina pectoris, arteriosclerosis, etc.).

The compounds that reduce the binding affinity between ligands and the G protein-coupled receptor protein of the present invention are useful as safe and low-toxic pharmaceuticals that decrease the physiological activities of ligands for the receptor protein of the present invention.

When compounds or their salt forms, which are obtainable by the screening methods or using the screening kits of the present invention, are employed as ingredients of the pharmaceuticals described above, the compounds can be formulated in the pharmaceuticals in a conventional manner. For example, the compounds can be prepared into tablets, capsules, elixir, microcapsules, aseptic solution, suspension, etc., as described for pharmaceuticals containing the receptor protein of the present invention.

The preparations thus obtained are safe and low-toxic, and can be administered to, for example, human and mammals (e.g., rats, rabbits, sheep, swine, bovine, cats, dogs, monkeys, etc.).

The dose of the compounds or their salt forms varies depending on subject to be administered, target organs, conditions, routes for administration, etc.; in oral administration, e.g., for the patient with suppression of eating, the dose is normally about 0.1 mg to about 100 mg, preferably about 1.0 to about 50 mg, and more preferably about 1.0 to about 20 mg per day (as 60 kg body weight). In parenteral administration, the single dose varies depending on subject to be administered, target organ, conditions, routes for administration, etc. but it is advantageous, e.g., for the patient with suppression of eating, to administer the active ingredient intravenously in a daily dose of about 0.01 to about 30 mg, preferably about 0.1 to about 20 mg, and more preferably about 0.1 to about 10 mg (as 60 kg body weight). For other animal species, the corresponding dose as converted per 60 kg body weight can be administered.

(8) Prophylactic and/or therapeutic agents for various diseases comprising the compounds (agonists or antagonists) that alter the binding property between the G protein-coupled receptor protein of the present invention and ligands

As described above, the receptor protein of the present invention may play some important role in the body such as a role in the central function. Therefore, the compounds (agonists or antagonists) that alter the binding property between the G protein-coupled receptor protein of the present invention and ligands can be used as prophylactic and/or therapeutic agents for diseases associated with dysfunction of the receptor protein of the present invention.

When the compounds are used as the prophylactic and/or therapeutic agents for diseases associated with dysfunction of the receptor protein of the present invention (e.g., central dysfunction (e.g., Alzheimer's disease, senile dementia, suppression of eating (anorexia), epilepsy, etc.), hormone diseases (e.g., weak pains, atonic bleeding, before and after expulsion, subinvolution of uterus, cesarean section, induced abortion, galactostasis, etc.), liver/gallbladder/pancreas/endocrine-associated diseases (e.g., diabetes mellitus, suppression of eating, etc.), inflammatory diseases (e. g., allergy, asthma, rheumatoid, etc.), circulatory diseases (e.g., hypertension, cardiac hypertrophy, angina pectoris, arteriosclerosis, etc.), the pharmaceutical preparations can be obtained in a conventional manner.

For example, the compounds can be administered orally as sugar coated tablet, capsule, elixir, and microcapsule, or non-orally as injection such as aseptic solution or suspension in water or other pharmaceutically acceptable liquid. For example, preparations of the compounds can be manufactured by mixing with physiologically acceptable known carrier, flavor, filler, vehicle, antiseptic, stabilizer, and binder in a unit-dosage form required for generally approved drug preparation. The amount of the active ingredient is set to an appropriate volume within the specified range.

For the additive that may be mixed in tablets, capsules, etc., for example, binders such as gelatin, cornstarch, tragacanth, and acacia, fillers such as crystalline cellulose, imbibers such as cornstarch, gelatin, and alginic acid, lubricants such as magnesium stearate, sweeteners such as sucrose and saccharin, and flavors such as peppermint, akamono oil and cherry are used. When the dosage form is a capsule, liquid carrier such as fat and oil may be contained. Aseptic compositions for injection can be formulated following the usual preparation such as dissolving or suspending the active substance in vehicle, e.g., water for injection, and natural plant oils e.g., sesame oil and coconut oil. For the aqueous solution for injection, for example, physiological saline and isotonic solutions (e.g., D-sorbitol, D-mannitol, sodium hydrochloride) containing glucose and other adjuvant are used. Appropriate dissolution-assisting agents, for example, alcohol (e.g., ethanol), polyalcohol (e.g., propylene glycol, polyethylene glycol), nonionic surfactant (e.g., polysorbate 80™, HCO-50) may be combined. For the oily solution, for example, sesame oil and soybean oil are used, and dissolution-assisting agents such as benzyl benzoate and benzyl alcohol may be combined.

The prophylactic/therapeutic agents described above may be combined, for example, with buffers (e. g., phosphate buffer, sodium acetate buffer), soothing agents (e.g., benzalkonium chloride, procaine hydrochloride), stabilizers (e.g., human serum albumin, polyethylene glycol), preservatives (e.g., benzyl alcohol, phenol), and antioxidants. The preparation for injection is usually filled in appropriate ampoules.

The preparations obtained as described above are safe and low toxic, and can be administered to, for example, human and mammals (e.g., rats, rabbits, sheep, swine, bovine, cats, dogs, monkeys, etc.).

The dose of the compounds or their salt forms varies depending on subject to be administered, target organs, conditions, routes for administration, etc.; in oral administration, e.g., for the patient with suppression of eating, the dose is normally about 0.1 mg to about 100 mg, preferably about 1.0 to about 50 mg, and more preferably about 1.0 to about 20 mg per day (as 60 kg body weight). In parenteral administration, the single dose varies depending on subject to be administered, target organ, conditions, routes for administration, etc. but it is advantageous, e.g., for the patient with suppression of eating, to administer the active ingredient intravenously in a daily dose of about 0.01 to about 30 mg, preferably about 0.1 to about 20 mg, and more preferably about 0.1 to about 10 mg (as 60 kg body weight). For other animal species, the corresponding dose as converted per 60 kg body weight can be administered.

(9) Quantification of the receptor protein of the present invention, its partial peptide, or its salt form

The antibodies of the present invention are capable of specifically recognizing the receptor protein of the present invention. Therefore, the antibodies can be used to quantify the receptor protein of the present invention in a test fluid, especially for quantification by the sandwich immunoassay. That is, the present invention provides, for example, the following quantification methods:

(i) a method of quantifying the receptor protein of the present invention in a test fluid, which comprises competitively reacting the antibody of the present invention with the test fluid and a labeled form of the receptor protein of the present invention, and measuring the ratio of the labeled receptor protein bound to the antibody; and,

(ii) a method of quantifying the receptor protein of the present invention in a test fluid, which comprises reacting the test fluid with the antibody of the present invention immobilized on a carrier and a labeled form of the antibody of the present invention simultaneously or sequentially, and measuring the activity of the label on the immobilized carrier.

In (ii) described above, it is preferred that one antibody recognizes the N-terminal region of the receptor protein of the present invention, and another antibody reacts with the C-terminal region of the receptor protein of the present invention.

Using monoclonal antibodies to the receptor protein of the present invention (hereinafter sometimes referred to as the monoclonal antibodies of the present invention), the receptor protein of the present invention can be assayed and also detected by tissue staining or the like. For this purpose, an antibody molecule itself may be used, or $F(ab')_2$, Fab' or Fab fractions of the antibody molecule may also be used. Assay methods using antibodies to the receptor protein of the present invention are not particularly limited. Any assay method can be used, so long as the amount of antibody, antigen, or antibody-antigen complex corresponding to the amount of antigen (e.g., the amount of the receptor protein) in the test fluid can be detected by chemical or physical means and the amount of the antigen can be calculated from a standard curve prepared from standard solutions containing known amounts of the antigen. For example, nephrometry, competitive methods, immunometric method, and sandwich method are appropriately used, with the sandwich method described below being most preferable in terms of sensitivity and specificity.

As the labeling agent for the methods using labeled substances, there are employed, for example, radioisotopes, enzymes, fluorescent substances, luminescent substances, etc. For the radioisotope, for example, [$^{125}$I], [$^{131}$I], [$^3$H] and [$^{14}$C] are used. As the enzyme described above, stable enzymes with high specific activity are preferred; for example, β-galactosidase, β-glucosidase, alkaline phosphatase, peroxidase, malate dehydrogenase and the like are used. Example of the fluorescent substance used are fluorescamine and fluorescein isothiocyanate are used. For the luminescent substance, for example, luminol, luminol derivatives, luciferin, and lucigenin. Furthermore, the biotin-avidin system may be used for binding antibody or antigen to the label.

For immobilization of antigen or antibody, physical adsorption may be used. Chemical binding methods conventionally used for insolubilization or immobilization of proteins or enzymes may also be used. For the carrier, for example, insoluble polysaccharides such as agarose, dextran, cellulose, etc.; synthetic resin such as polystyrene, polyacrylamide, silicon, etc., and glass or the like. are used.

In the sandwich method, the immobilized monoclonal antibody of the present invention is reacted with a test fluid (primary reaction), then with the labeled monoclonal antibody of the present invention (secondary reaction), and the activity of the label on the immobilizing carrier is measured, whereby the amount of the receptor protein of the present invention in the test fluid can be quantified. The order of the primary and secondary reactions may be reversed, and the reactions may be performed simultaneously or with an interval. The methods of labeling and immobilization can be performed by the methods described above.

In the immunoassay by the sandwich method, the antibody used for immobilized or labeled antibodies is not necessarily one species, but a mixture of two or more species of antibody may be used to increase the measurement sensitivity.

In the methods of assaying the receptor protein of the present invention by the sandwich method, antibodies that bind to different sites of the receptor protein are preferably used as the monoclonal antibodies of the present invention for the primary and secondary reactions. That is, in the antibodies used for the primary and secondary reactions are, for example, when the antibody used in the secondary reaction recognizes the C-terminal region of the receptor protein, it is preferable to use the antibody recognizing the region other than the C-terminal region for the primary reaction, e.g., the antibody recognizing the N-terminal region.

The monoclonal antibodies of the present invention can be used for the assay systems other than the sandwich method, for example, competitive method, immunometric method, nephrometry, etc. In the competitive method, antigen in a test fluid and the labeled antigen are competitively reacted with antibody, and the unreacted labeled antigen (F) and the labeled antigen bound to the antibody (B) are separated (B/F separation). The amount of the label in B or F is measured, and the amount of the antigen in the test fluid is quantified. This reaction method includes a liquid phase method using a soluble antibody as an antibody, polyethylene glycol for B/F separation and a secondary antibody to the soluble antibody, and an immobilized method either using an immobilized antibody as the primary antibody, or using a soluble antibody as the primary antibody and immobilized antibody as the secondary antibody.

In the immunometric method, antigen in a test fluid and immobilized antigen are competitively reacted with a definite amount of labeled antibody, the immobilized phase is separated from the liquid phase, or antigen in a test fluid and an excess amount of labeled antibody are reacted, immobilized antigen is then added to bind the unreacted labeled antibody to the immobilized phase, and the immobilized phase is separated from the liquid phase. Then, the amount of the label in either phase is measured to quantify the antigen in the test fluid.

In the nephrometry, insoluble precipitate produced after the antigen-antibody reaction in gel or solution is quantified. When the amount of antigen in the test fluid is small and only a small amount of precipitate is obtained, laser nephrometry using scattering of laser is advantageously employed.

For applying these immunological methods to the measurement methods of the present invention, any particular conditions or procedures are not required. Systems for measuring the receptor protein of the present invention or its salts are constructed by adding the usual technical consideration in the art to the conventional conditions and procedures. For the details of these general technical means, reference can be made to the following reviews and texts. [For example, Hiroshi Irie, ed. "Radioimmunoassay" (Kodansha, published in 1974), Hiroshi Irie, ed. "Sequel to the Radioimmunoassay" (Kodansha, published in 1979), Eiji Ishikawa, et al. ed. "Enzyme immonoassay" (Igakushoin, published in 1978), Eiji Ishikawa, et al. ed. "Immunoenzyme assay" (2nd ed.) (Igakushoin, published in 1982), Eiji Ishikawa, et al. ed. "Immunoenzyme assay" (3rd ed.) (Igakushoin, published in 1987), Methods in ENZYMOLOGY, Vol. 70 (Immunochemical Techniques (Part A)), ibid., Vol. 73 (Immunochemical Techniques (Part B)), ibid., Vol. 74 (Immunochemical Techniques (Part C)), ibid., Vol. 84 (Immunochemical Techniques (Part D: Selected Immunoassays)), ibid., Vol. 92 (Immunochemical Techniques (Part E: Monoclonal Antibodies and General Immunoassay Methods)), ibid., Vol. 121 (Immunochemical Techniques (Part I: Hybridoma Technology and Monoclonal Antibodies))(all published by Academic Press Publishing).

As described above, the receptor protein of the present invention or its salts can be quantified with high sensitivity, using the antibodies of the present invention.

By quantifying the receptor protein of the present invention or its salts in vivo using the antibodies of the present invention, diagnosis can be made on various diseases associated with dysfunction of the receptor protein of the present invention (e.g., central dysfunction (e.g., Alzheimer's disease, senile dementia, suppression of eating (anorexia), epilepsy, etc.), hormone diseases (e.g., weak pains, atonic bleeding, before and after expulsion, subinvolution of uterus, cesarean section, induced abortion, galactostasis, etc.), liver/gallbladder/pancreas/endocrine-associated diseases (e.g., diabetes mellitus, suppression of eating, etc.), inflammatory diseases (e.g., allergy, asthma, rheumatoid, etc.), circulatory diseases (e.g., hypertension, cardiac hypertrophy, angina pectoris, arteriosclerosis, etc.).

The antibodies of the present invention can also be used for specifically detecting the receptor protein of the present invention present in test samples such as body fluids or tissues. The antibodies may also be used for preparation of antibody columns for purification of the receptor protein of the present invention, for detection of the receptor protein of the present invention in each fraction upon purification, and for analysis of the behavior of the receptor protein of the present invention in the test cells.

(10) Methods of screening compounds that alter the amount of the receptor protein of the present invention or its partial peptide in cell membranes

Since the antibodies of the present invention specifically recognize the receptor protein, its partial peptide, or its salt of the present invention, the antibodies can be used for screening of the compounds that alter the amount of the receptor protein of the present invention or its partial peptide in cell membranes.

That is, the present invention provides, for example, the following methods:

(i) a method of screening compounds that alter the amount of the receptor protein of the present invention or its partial peptides in cell membranes, which comprises disrupting ① blood, ② specific organs, ③ tissues or cells isolated from the organs of non-human mammals, isolating the cell membrane fraction and then quantifying the receptor protein of the present invention or its partial peptide contained in the cell membrane fraction;

(ii) a method of screening compounds that alter the amount of the receptor protein of the present invention or its partial peptides in cell membranes, which comprises disrupting transformants, etc. expressing the receptor protein of the present invention or its partial peptides , isolating the cell membrane fraction, and then quantifying the receptor protein of the present invention or its partial peptides contained in the cell membrane fraction;

(iii) a method of screening compounds that alter the amount of the receptor protein of the present invention or its partial peptides in cell membranes, which comprises sectioning ① blood, ② specified organs, ③ tissues or cells isolated from the organs of non-human mammals, immunostaining, and then quantifying the staining intensity of the receptor protein in the cell surface layer to confirm the protein on the cell membrane; and,

(iv) a method of screening compounds that alter the amount of the receptor protein of the present invention or its partial peptides in cell membranes, which comprises sectioning transformants, etc. expressing the receptor protein of the present invention or its partial peptides , immunostaining, and then quantifying the staining intensity of the receptor protein in the cell surface layer to confirm the protein on the cell membrane.

Specifically, the receptor protein and its partial peptides of the present invention contained in cell membrane fractions are quantified as follows.

(i) Normal or non-human mammals of disease models (e.g., mice, rats, rabbits, sheep, swine, bovine, cats, dogs, monkeys, more specifically, rats with dementia, obese mice, rabbits with arteriosclerosis, tumor-bearing mice, etc.) are administered with a drug (e.g., anti-dementia agents, hypotensive agents, anticancer agents, antiobestic agents) or physical stress (e.g., soaking stress, electric shock, light and darkness, low temperature, etc.), and the blood, specific organs (e.g., brain, liver, kidneys), or tissue or cells isolated from the organs are obtained after a specified period of time. The obtained organs, tissues or cells are suspended in, for example, an appropriate buffer (e.g., Tris hydrochloride buffer, phosphate buffer, Hepes buffer), and the organs, tissues, or cells are disrupted, and the cell membrane fraction is obtained using surfactants (e. g., Triton-X 100™, Tween 20™) and further using techniques such as centrifugal separation, filtration, and column fractionation.

The cell membrane fraction refers to a fraction abundant in cell membrane obtained by cell disruption and subsequent fractionation by a publicly known method. Useful cell disruption methods include cell squashing using a Potter-Elvehjem homogenizer, disruption using a Waring blender or Polytron (manufactured by Kinematica Inc.), disruption by ultrasonication, and disruption by cell spraying through thin nozzles under an increased pressure using a French press or the like. Cell membrane fractionation is effected mainly by fractionation using a centrifugal force, such as centrifugation for fractionation and density gradient centrifugation. For example, cell disruption fluid is centrifuged at a low speed (500 rpm to 3,000 rpm) for a short period of time (normally about 1 to about 10 minutes), the resulting supernatant is then centrifuged at a higher speed (15,000 rpm to 30,000 rpm) normally for 30 minutes to 2 hours. The precipitate thus obtained is used as the membrane fraction. The membrane fraction is rich in the receptor protein expressed and membrane components such as cell-derived phospholipids and membrane proteins.

The receptor protein of the present invention or its partial peptides contained in the cell membrane fraction can be quantified by, for example, the sandwich immunoassay and western blot analysis using the antibodies of the present invention.

The sandwich immunoassay can be performed as described above, and the western blot can be performed by publicly known methods.

(ii) Transformants expressing the receptor protein of the present invention or its partial peptides are prepared following the method described above, and the receptor protein of the present invention or its partial peptides contained in the cell membrane fraction can be quantified.

The compounds that alter the amount of the receptor protein of the present invention or its partial peptides in cell membranes can be screened as follows.

(i) To normal or disease models of non-human mammals, a test compound is administered at a specified 'period of time before (30 minutes to 24 hours before, preferably 30 minutes to 12 hours before, more preferably 1 hour to 6 hours before), at a specified time after (30 minutes to 3 days after, preferably 1 hour to 2 days after, more preferably 1 hour to 24 hours after), or simultaneously with a drug or physical stress. At a specified time (30 minute to 3 days, preferably 1 hour to 2 days, more preferably 1 hour to 24 hours) after administration of the test compound, the amount of the receptor protein of the present invention or its partial peptides contained in cell membranes are quantified.

(ii) Transformants are cultured in a conventional manner and a test compound is mixed in the culture medium. After a specified time (after 1 day to 7 days, preferably after 1 day to 3 days, more preferably after 2 to 3 days), the amount of the receptor protein of the present invention or its partial peptides contained in the cell membranes can be quantified.

Specifically, the receptor protein of the present invention or its partial peptides contained in cell membrane fractions are confirmed as follows.

(iii) Normal or non-human mammals of disease models (e.g., mice, rats, rabbits, sheep, swine, bovine, cats, dogs, monkeys, more specifically, rats with dementia, obese mice, rabbits with arteriosclerosis, tumor-bearing mice, etc.) are administered with a drug (e.g., anti-dementia agents, hypotensive agents, anticancer agents, antiobestic agents) or physical stress (e.g., soaking stress, electric shock, light and darkness, low temperature, etc.), and the blood, specific organs (e.g., brain, liver, kidneys), or tissue or cells isolated from the organs are obtained after a specified period of time. Tissue sections are prepared from the thus obtained organs, tissues or cells in a conventional manner followed by immunostaining with the antibody of the present invention. The staining intensity of the receptor protein in the cell surface layer is quantified to confirm the protein on the cell membrane, the amount of the receptor protein of the present invention or its partial peptides in the cell membrane can be quantitatively or qualitatively confirmed.

(iv) The confirmation can also be made by the similar method, using transformants expressing the receptor protein of the present invention or its partial peptides .

The compounds or its salts, which is obtainable by the screening methods of the present invention, are the compounds that alter the amount of the receptor protein or its peptide fragments of the present invention. Specifically, these compounds are; (a) compounds that potentiate the G protein-coupled receptor-mediated cell-stimulating activity (e.g., activity that promotes or inhibits arachidonic acid release, acetylcholine release, intracellular $Ca^{2+}$ release, intracellular cAMP production, intracellular cGMP production, inositol phosphate production, changes in cell membrane potential, phosphorylation of intracellular proteins, activation of c-fos, pH reduction, etc.) (so-called agonists to the receptor protein of the present invention), by increasing thea mount of the receptor protein of the present invention or its partial peptides ; and (b) compounds that lower the cell stimulating-activity by decreasing the amount of the receptor protein of the present invention.

The compounds may be peptides, proteins, non-peptide compounds, synthetic compounds, fermentation products, and may be novel or known compounds.

The compounds that increase the cell-stimulating activity are useful as safe and low-toxic pharmaceuticals for potentiation of the physiological activity of the receptor protein of the present invention (for the prevention and/or treatment of central dysfunction (e.g., Alzheimer's disease, senile dementia, suppression of eating (anorexia), epilepsy, etc.), hormone diseases (e. g., weak pains, atonic bleeding, before and after expulsion, subinvolution of uterus, cesarean section, induced abortion, galactostasis, etc.), liver/gallbladder/pancreas/endocrine-associated diseases (e.g., diabetes mellitus, suppression of eating, etc.), inflammatory diseases (e.g., allergy, asthma, rheumatoid, etc.), circulatory diseases (e.g., hypertension, cardiac hypertrophy, angina pectoris, arteriosclerosis, etc.).

The compounds that decrease the cell-stimulating activity are useful as safe and low-toxic pharmaceuticals for reduction of the physiological activity of the receptor protein of the present invention.

When compounds or their salt forms, which are obtainable by the screening methods of the present invention, are used as for pharmaceutical compositions, preparations can be prepared following the conventional methods. For example, as described above for preparation of the pharmaceuticals containing the receptor protein of the present invention, the compounds can be prepared into tablets, capsules, elixir, microcapsules, aseptic solution, suspension, etc.

Since the preparations thus obtained are safe and low-toxic, the preparations can be administered to human or mammals (e.g., rats, rabbits, sheep, swine, bovine, cats, dogs, monkeys, etc.).

The dose of the compounds or their salt forms varies depending on subject to be administered, target organs, conditions, routes for administration, etc.; in oral administration, e.g., for the patient with suppression of eating, the dose is normally about 0.1 mg to about 100 mg, preferably about 1.0 to about 50 mg, and more preferably about 1.0 to about 20 mg per day (as 60 kg body weight). In parenteral administration, the single dose varies depending on subject to be administered, target organ, conditions, routes for administration, etc. but it is advantageous, e.g., for the patient with suppression of eating, to administer the active ingredient intravenously in a daily dose of about 0.01 to about 30 mg, preferably about 0.1 to about 20 mg, and more preferably about 0.1 to about 10 mg (as 60 kg body weight). For other animal species, the corresponding dose as converted per 60 kg body weight can be administered.

(11) Prophylactic and/or therapeutic agents for various diseases comprising compounds that alter the amount of the receptor protein of the present invention or its partial peptides in cell membrane

As described above, the receptor protein of the present invention is considered to play some important role in vivo, such as a role in the central function. Therefore, the compounds that alter the amount of the receptor protein of the present invention or its partial peptide in cell membrane can be used as prophylactic and/or therapeutic agents for diseases associated with dysfunction of the receptor protein of the present invention.

When the compounds are used as prophylactic and/or therapeutic agents for diseases associated with dysfunction of the receptor protein of the present 'invention, the preparations can be obtained in a conventional manner.

For example, the compounds can be administered orally as a sugar coated tablet, capsule, elixir, and microcapsule, or parenterally as injection such as aseptic solution and suspension in water or other pharmaceutically acceptable liquid. For example, preparations of the compounds can be manufactured by mixing with physiologically acceptable known carrier, flavor, filler, vehicle, antiseptic, stabilizer, and binder in a unit-dosage form required for generally approved drug preparation. The amount of the active ingredient is set to an appropriate volume within the specified range.

For the additive that may be mixed in tablets and capsules, for example, binders such as gelatin, cornstarch, tragacanth, and acacia, fillers such as crystalline cellulose, imbibers such as cornstarch, gelatin, and alginic acid, lubricants such as magnesium stearate, sweeteners such as sucrose and saccharin, and flavors such as peppermint, akamono oil and cherry are used. When the dosage form is a capsule, liquid carrier such as fat and oil may be contained. Aseptic compositions for injection can be formulated following the usual preparation such as dissolving or suspending the active substance in vehicle, e.g., water for injection, and natural plant oils e.g., sesame oil and coconut oil. For the aqueous solution for injection, for example, physiological saline and isotonic solutions (e.g., D-sorbitol, D-mannitol, sodium hydrochloride) containing glucose and other adjuvant are used. Appropriate dissolution-assisting agents, for example, alcohol (e.g., ethanol), polyalcohol (e.g., propylene glycol, polyethylene glycol), nonionic surfactant (e.g., polysorbate 80™, HCO-50) may be combined. For the oily solution, for example, sesame oil and soybean oil are used, and dissolution-assisting agents such as benzyl benzoate and benzyl alcohol may be combined.

The prophylactic/therapeutic agents described above may be combined with buffers (e.g., phosphate buffer, sodium acetate buffer), soothing agents (e.g., benzalkonium chloride, procaine hydrochloride), stabilizers (e.g., human serum albumin, polyethylene glycol), preservatives (e.g., benzyl alcohol, phenol), and antioxidants. The preparation for injection is usually filled in appropriate ampoules.

Since the preparations thus obtained are safe and low toxic, the preparation can be administered to, for example, human and mammals (e.g., rats, rabbits, sheep, swine, bovine, cats, dogs, monkeys, etc.).

The dose of the compounds or their salt forms varies depending on subject to be administered, target organs, conditions, routes for administration, etc.; in oral administration, e.g., for the patient with suppression of eating, the dose is normally about 0.1 mg to about 100 mg, preferably about 1.0 to about 50 mg, and more preferably about 1.0 to about 20 mg per day (as 60 kg body weight). In parenteral administration, the single dose varies depending on subject to be administered, target organ, conditions, routes for administration, etc. but it is advantageous, e.g., for the patient with suppression of eating, to administer the active ingredient intravenously in a daily dose of about 0.01 to about 30 mg, preferably about 0.1 to about 20 mg, and more preferably about 0.1 to about 10 mg (as 60 kg body weight). For other animal species, the corresponding dose as converted per 60 kg body weight can be administered.

(12) Neutralization with antibodies to the receptor protein, its partial peptides, or its salts of the present invention

The neutralizing activity of antibodies to the receptor protein of the present invention, its partial peptides, or its salts refer to an activity of inactivating the signal transduction function involving the receptor protein. Therefore, when the antibody has the neutralizing activity, the antibody can inactivate the signal transduction in which the receptor protein participates, for example, inactivate the receptor protein-mediated cell-stimulating activity (e.g., activity that promotes or inhibits arachidonic acid release, acetylcholine release, intracellular Ca$^{2+}$ release, intracellular CAMP production, intracellular cGMP production, inositol phosphate production, changes in cell membrane potential, phosphorylation of intracellular proteins, activation of c-fos, pH reduction, etc.). Therefore, the antibody can be used for the prevention and/or treatment of diseases caused by overexpression of the receptor protein.

(13) Preparation of non-human animals carrying the DNA encoding the G protein-coupled receptor protein of the present invention

Using the DNA of the present invention, non-human transgenic animals expressing the receptor protein of the present invention can be prepared. Examples of the non-human animals include mammals (e.g., rats, mice, rabbits, sheep, swine, bovine, cats, dogs, monkeys, etc.) (hereinafter merely referred to as animals) can be used, with mice and rabbits being particularly appropriate.

To transfer the DNA of the present invention to target animals, it is generally advantageous to use the DNA in a gene construct ligated downstream of a promoter that can express the DNA in animal cells. For example, when the DNA of the present invention derived from rabbit is transferred, e.g., the gene construct, in which the DNA is ligated downstream of a promoter that can expresses the DNA of the present invention derived from animals containing the DNA of the present invention highly homologous to the rabbit-derived DNA, is microinjected to rabbit fertilized ova; thus, the DNA-transferred animal, which is capable of producing a high level of the receptor protein of the present invention, can be produced. Examples of the promoters that are usable include virus-derived promoters and ubiquitous expression promoters such as metallothionein promoter, but promoters of NGF gene and enolase that are specifically expressed in the brain are preferably used.

The transfer of the DNA of the present invention at the fertilized egg cell stage secures the presence of the DNA in all germ and somatic cells in the produced animal. The presence of the receptor protein of the present invention in the germ cells in the DNA-transferred animal means that all germ and somatic cells contain the receptor protein of the present invention in all progenies of the animal. The progenies of the animal that took over the gene contain the receptor protein of the present invention in all germ and somatic cells.

The DNA-transferred animals of the present invention can be maintained and bled in the conventional environment as animals carrying the DNA after confirming the stable retention of the gene in the animals through mating. Furthermore, mating male and female animals containing the objective DNA results in acquiring homozygote animals having the transferred gene on both homologous chromosomes. By mating the male and female homozygotes, bleeding can be performed so that all progenies contain the DNA.

Since the receptor protein of the present invention is highly expressed in the animals in which the DNA of the present invention has been transferred, the animals are useful for screening of agonists or antagonists to the receptor protein of the present invention.

The animals in which the DNA of the present invention has been transferred can also be used as cell sources for tissue culture. The receptor protein of the present invention can be analyzed by, for example, directly analyzing the DNA or RNA in tissues from the mouse in which the DNA of the present invention has been transferred, or by analyzing tissues containing the receptor protein expressed from the gene. Cells from tissues containing the receptor protein of the present invention are cultured by the standard tissue culture technique. Using these cells, for example, the function of tissue cells such as cells derived from the brain or peripheral tissues, which are generally difficult to culture, can be studied. Using these cells, for example, it is possible to select pharmaceuticals that increase various tissue functions. When a highly expressing cell line is available, the receptor protein of the present invention can be isolated and purified from the cell line.

In the specification and drawings, the codes of bases and amino acids are denoted in accordance with the IUPAC-IUB Commission on Biochemical Nomenclature or by the common codes in the art, examples of which are shown below. For amino acids that may have the optical isomer, L form is presented unless otherwise indicated.

DNA : deoxyribonucleic acid
cDNA : complementary deoxyribonucleic acid
A : adenine
T : thymine
G : guanine
C : cytosine
RNA : ribonucleic acid
mRNA : messenger ribonucleic acid
dATP : deoxyadenosine triphosphate
dTTP : deoxythymidine triphosphate
dGTP : deoxyguanosine triphosphate
dCTP : deoxycytidine triphosphate
ATP : adenosine triphosphate
EDTA : ethylenediaminetetraacetic acid
SDS : sodium dodecyl sulfate
Gly : glycine
Ala : alanine
Val : valine
Leu : leucine
Ile : isoleucine
Ser : serine
Thr : threonine
Cys : cysteine
Met : methionine
Glu : glutamic acid
Asp : aspartic acid

Lys : lysine
Arg : arginine
His : histidine
Phe : phenylalanine
Tyr : tyrosine
Trp : tryptophan
Pro : proline
Asn : asparagine
Gln : glutamine
pGlu : pyroglutamic acid
Me : methyl
Et : ethyl
Bu : butyl
Ph : phenyl
TC : thiazolidine-4(R)-carboxamide

The substituents, protective groups and reagents, which are frequently used throughout the specification, are shown by the following abbreviations.

Tos : p-toluenesulfonyl
CHO : formyl
Bzl : benzyl
Cl$_2$Bl: 2,6-dichlorobenzyl
Bom : benzyloxymethyl
Z : benzyloxycarbonyl
Cl-Z : 2-chlorobenzyloxycarbonyl
Br-Z : 2-bromobenzyloxycarbonyl
Boc : t-butoxycarbonyl
DNP : dinitrophenol
Trt : trityl
Bum : t-butoxymethyl
Fmoc : N-9-fluorenylmethoxycarbonyl
HOBt : 1-hydroxybenztriazole
HOOBt: 3,4-dihydro-3-hydroxy-4-oxo-1,2,3-benzotriazine
HONB : 1-hydroxy-5-norbornene-2,3-dicarboximide
DCC : N,N'-dicyclohexylcarbodiimide

The sequence identification numbers in the sequence listing of the specification indicates the following sequence, respectively.

[SEQ ID NO:1]

This shows the amino acid sequence of human hippocampus-derived, novel G protein-coupled receptor protein hSLT of the present invention.

[SEQ ID NO:2]

This shows the base sequence of cDNA encoding human hippocampus-derived, novel G protein-coupled receptor protein hSLT of the present invention, which has the amino acid sequence shown by SEQ ID NO:1.

[SEQ ID NO:3]

This shows the base sequence of primer 1 used for cloning cDNA encoding human hippocampus-derived, novel G protein-coupled receptor protein hSLT of the present invention.

[SEQ ID NO:4]

This shows the base sequence of primer 2 used for cloning cDNA encoding human hippocampus-derived, novel G protein-coupled receptor protein hSLT of the present invention.
Escherichia coli DH5α/pCR3.1-hSLT obtained in Example 1 later described was on deposit with the Ministry of International Trade and Industry, Agency of Industrial Science and Technology, National Institute of Bioscience and Human Technology (NIBH), located at 1-1-3, Higashi, Tsukuba-shi, Ibaraki, Japan, as the Accession Number FERM BP-6710 on April 28, 1999 and with Institute for Fermentation (IFO), located at 2-17-85, Juso Honcho, Yodogawa-ku, Osaka-shi, Osaka, Japan, as the Accession Number IFO 16284 on April 20, 1999.

EXAMPLES

The present invention is described in detail below with reference to REFERENCE EXAMPLES and EXAMPLES, but is not deemed to limit the scope of the present invention thereto. The gene manipulation procedures using Escherichia coli were performed according to the methods described in the Molecular Cloning.

EXAMPLE 1

Cloning of the cDNA encoding the human hippocampus-derived G protein-coupled receptor protein and determination of the base sequence
Using human hippocampus-derived cDNA (CLONTECH Inc.) as a template and two primers, namely, primer 1 (SEQ ID NO:3) and primer 2 (SEQ ID NO:4), PCR was carried out. The reaction solution in the above reaction comprised of 1/10 volume of the cDNA, 1/50 volume of Advantage 2 Polymerase Mix (CLONTECH Inc.), 0.2 μM of primer 1 (SEQ ID NO:3), 0.2 μM of primer 2 (SEQ ID NO:4), 200 μM of dNTPs and a buffer attached to the enzyme to make the final volume 25 μl. The PCR was carried out by cycles of (1) 94°C for 1 minute, (2) then a cycle set to include 94°C for 20 seconds followed by 72°C for 2 minutes, which was repeated 3 times, (3) a cycle set to include 94°C for 20 seconds followed by 65°C for 20 seconds and 68°C for 2 minutes, which was repeated 3 times, (4) a cycle set to include 94°C for 20 seconds followed by 58°C for 20 seconds and 68°C for 2 minutes, which was repeated 36 times, and (3) finally, extension reaction at 68°C for 7 minutes. After completion of the PCR reaction, the product was subcloned to plasmid vector pCR3.1 (Invitrogen Inc.)

following the instructions attached to the TA cloning kit (Invitrogen Inc.), which was then introduced into Escherichia coli DH5$\alpha$, and the clones containing the cDNA were selected on LB agar plates containing ampicillin. The sequence of each clone was analyzed to give the cDNA sequence (SEQ ID NO :2) encoding the novel G protein-coupled receptor protein. The novel G protein-coupled receptor protein containing the, amino acid sequence (SEQ ID NO:1) deduced therefrom was designated hSLT and the transformant was designated Escherichia coli DH5$\alpha$/pCR3.1-hSLT.

EXAMPLE 2 Preparation of hSLT-expressing CHO cells

Using plasmid pCR3.1-hSLT bearing a gene encoding the full-length amino acid sequence of human hippocampus-derived hSLT, which sequence was confirmed in EXAMPLE 1, E. coli DH5$\alpha$ (Toyobo) was transformed, the transformants were cultured, and the plasmid DNA of pCR3.1-hSLT was prepared using Plasmid Midi kit (Qiagen Inc.). The plasmid DNA was inserted into CHO-K1 cells, using CellPhect Transfection Kit (Amersham Pharmacia Biotech Inc.) following the protocol attached to the kit. Ten mg of the plasmid DNA was co-precipitated with calcium phosphate in suspension, and charged in a 10 cm dish, in which $5 \times 10^5$ or $1 \times 10^6$ CHO-K1 cells were seeded before 24 hours, and the cells were cultured in DMEM medium containing 10% fetal calf serum for one day. After passage, the cells were cultured in DMEM medium containing 1 mg/ml G418. From the colonies of hSLT-expressing CHO cells growing in the selection medium,40 clones were selected. After the entire RNA was extracted from further selected hSLT-expressing CHO cells in a conventional manner, the mRNA level of hSLT was assayed by the TaqMan method to determine the number of copies. As the result, 3 clones (#5, #7 and #28) were selected as having a high expression level.

INDUSTRIAL APPLICABILITY

The G protein-coupled receptor protein of the present invention, its partial peptides, or salts thereof and the polynucleotides encoding the same (e.g. DNA, RNA, and its derivatives) can be used for; ① determination of ligands (agonists); ② preparation of antibodies and antisera; ③ construction of recombinant receptor protein expression systems; ④ development of the receptor binding assay systems using the expression systems and screening of pharmaceutical candidate compounds; ⑤ effecting drug design based on comparison with structurally similar ligand receptors; ⑥ reagents for preparation of probes and PCR primers for gene diagnosis; ⑦ production of transgenic animals; and ⑧ pharmaceutical drugs for the gene prophylaxis and gene therapy.

## Claims

1. A protein containing the same or substantially the same amino acid sequence as the amino acid sequence represented by SEQ ID NO:1, or a salt thereof.

2. A partial peptide of the protein according to claim 1, or a salt thereof.

3. A polynucleotide containing a polynucleotide having a base sequence encoding the protein according to claim 1.

4. A polynucleotide according to claim 3, which is DNA.

5. A polynucleotide according to claim 3, which is represented by SEQ ID NO:2.

6. A recombinant vector containing the polynucleotide according to claim 3.

7. A transformant transformed with the recombinant vector according to claim 6.

8. A method of manufacturing the protein or its salt according to claim 1, which comprises culturing the transformant according to claim 7 and accumulating the protein according to claim 1.

9. An antibody to the protein according to claim 1, the partial peptide according to claim 3, or a salt of the protein or partial peptide.

10. An antibody according to claim 9, which is a neutralizing antibody capable of inactivating signal transduction of the protein according to claim 1.

11. A diagnostic composition comprising the antibody according to claim 9.

12. A ligand to the protein or its salt according to claim 1, which is obtainable using the protein according to claim 1 or the partial peptide according to claim 1, or a salt of said protein or partial peptide.

13. A pharmaceutical composition comprising the ligand according to claim 12.

14. A method of determining a ligand to the protein or its salt according to claim 1, which comprises using the protein according to claim 1 or the partial peptide according to claim 1, or a salt of said protein or partial peptide.

15. A method of screening a compound that alters the binding property between a ligand and the protein or its salt according to claim 1, wherein the protein according to claim 1, the partial peptide according to claim 1, or a salt of the protein or partial peptide.

16. A kit for screening a compound or its salt that alters the binding property between a ligand and the protein or its salt according to claim 1, comprising the protein according to claim 1 or the partial peptide according to claim 1, or a salt of said protein or partial peptide.

17. A compound or its salt that alters the binding property between a ligand and the protein or its salt according to claim 1, which is obtainable using the screening method according to claim 15 or the screening kit according to claim 16.

18. A pharmaceutical composition comprising a compound or its salt that alters the binding property between a ligand and the protein or its salt according to claim 1, which is obtainable using the screening method according to claim 15 or the screening kit according to claim 16.

19. A polynucleotide that hybridizes to the polynucleotide according to claim 3 under a highly stringent condition.

20. A polynucleotide comprising a base sequence complementary to the polynucleotide according to claim 3 or a part of the base sequence.

21. A method of quantifying mRNA of the protein according to claim 1, which comprises using the polynucleotide according to claim 3 or a part of the polynucleotide.

22. A method of quantifying the protein according to claim 1, which comprises using the antibody according to claim 9.

23. A diagnostic method for a disease associated with functions of the protein according to claim 1, which comprises using the quantification method according to claim 21 or claim 22.

24. A method of screening a compound or its salt that alters the expression level of the protein according to claim 1, which comprises using the quantification method according to claim 21.

25. A method of screening a compound or its salt that alters the amount of the protein according to claim 1 in cell membrane, which comprises using the quantification method according to claim 22.

26. A compound or its salt that alters the expression level of the protein according to claim 1, 'which is obtainable using the screening method according to claim 24.

27. A compound or its salt that alters the amount of the protein according to claim 1 in cell membrane, which is obtainable using the screening method according to claim 25.

# Fig 1

```
        10          20          30          40          50          60
ATGAATCCAT TTCATGCATC TTGTTGGAAC ACCTCTGCCG AACTTTTAAA CAAATCCTGG
METAsnProP heHisAlaSe rCysTrpAsn ThrSerAlaG luLeuLeuAs nLysSerTrp


        70          80          90         100         110         120
AATAAAGAGT TTGCTTATCA AACTGCCAGT GTGGTAGATA CAGTCATCCT CCCTTCCATG
AsnLysGluP heAlaTyrGl nThrAlaSer ValValAspT hrValIleLe uProSerMET


       130         140         150         160         170         180
ATTGGGATTA TCTGTTCAAC AGGGCTGGTT GGCAACATCC TCATTGTATT CACTATAATA
IleGlyIleI leCysSerTh rGlyLeuVal GlyAsnIleL euIleValPh eThrIleIle


       190         200         210         220         230         240
AGATCCAGGA AAAAAACAGT CCCTGACATC TATATCTGCA ACCTGGCTGT GGCTGATTTG
ArgSerArgL ysLysThrVa lProAspIle TyrIleCysA snLeuAlaVa lAlaAspLeu


       250         260         270         280         290         300
GTCCACATAG TTGGAATGCC TTTTCTTATT CACCAATGGG CCCGAGGGGG AGAGTGGGTG
ValHisIleV alGlyMETPr oPheLeuIle HisGlnTrpA laArgGlyGl yGluTrpVal


       310         320         330         340         350         360
TTTGGGGGGC CTCTCTGCAC CATCATCACA TCCCTGGATA CTTGTAACCA ATTTGCCTGT
PheGlyGlyP roLeuCysTh rIleIleThr SerLeuAspT hrCysAsnGl nPheAlaCys


       370         380         390         400         410         420
AGTGCCATCA TGACTGTAAT GAGTGTGGAC AGGTACTTTG CCCTCGTCCA ACCATTTCGA
SerAlaIleM ETThrValME TSerValAsp ArgTyrPheA laLeuValGl nProPheArg


       430         440         450         460         470         480
CTGACACGTT GGAGAACAAG GTACAAGACC ATCCGGATCA ATTTGGGCCT TTGGGCAGCT
LeuThrArgT rpArgThrAr gTyrLysThr IleArgIleA snLeuGlyLe uTrpAlaAla


       490         500         510         520         530         540
TCCTTTATCC TGGCATTGCC TGTCTGGGTC TACTCGAAGG TCATCAAATT TAAAGACGGT
SerPheIleL euAlaLeuPr oValTrpVal TyrSerLysV alIleLysPh eLysAspGly
```

# Fig 2

```
          550        560        570        580        590        600
GTTGAGAGTT GTGCTTTTGA TTTGACATCC CCTGACGATG TACTCTGGTA TACACTTTAT
ValGluSerC ysAlaPheAs pLeuThrSer ProAspAspV alLeuTrpTy rThrLeuTyr


          610        620        630        640        650        660
TTGACGATAA CAACTTTTTT TTTCCCTCTA CCCTTGATTT TGGTGTGCTA TATTTTAATT
LeuThrIleT hrThrPhePh ePheProLeu ProLeuIleL euValCysTy rIleLeuIle


          670        680        690        700        710        720
TTATGCTATA CTTGGGAGAT GTATCAACAG AATAAGGATG CCAGATGCTG CAATCCCAGT
LeuCysTyrT hrTrpGluME TTyrGlnGln AsnLysAspA laArgCysCy sAsnProSer


          730        740        750        760        770        780
GTACCAAAAC AGAGAGTGAT GAAGTTGACA AAGATGGTGC TGGTGCTGGT GGTAGTCTTT
ValProLysG lnArgValME TLysLeuThr LysMETValL euValLeuVa lValValPhe


          790        800        810        820        830        840
ATCCTGAGTG CTGCCCCTTA TCATGTGATA CAACTGGTGA ACTTACAGAT GGAACAGCCC
IleLeuSerA laAlaProTy rHisValIle GlnLeuValA snLeuGlnME TGluGlnPro


          850        860        870        880        890        900
ACACTGGCCT TCTATGTGGG TTATTACCTC TCCATCTGTC TCAGCTATGC CAGCAGCAGC
ThrLeuAlaP heTyrValGl yTyrTyrLeu SerIleCysL euSerTyrAl aSerSerSer


          910        920        930        940        950        960
ATTAACCCTT TTCTCTACAT CCTGCTGAGT GGAAATTTCC AGAAACGTCT GCCTCAAATC
IleAsnProP heLeuTyrIl eLeuLeuSer GlyAsnPheG lnLysArgLe uProGlnIle


          970        980        990       1000       1010       1020
CAAAGAAGAG CGACTGAGAA GGAAATCAAC AAATATGGAA ACACTCTGAA ATCACACTTT
GlnArgArgA laThrGluLy sGluIleAsn AsnMETGlyA snThrLeuLy sSerHisPhe


         1030
TAG
‡‡‡
```

# Fig 3

<table>
<tr><td colspan="2" align="center"><strong>INTERNATIONAL SEARCH REPORT</strong></td><td>International application No.<br><br>PCT/JP00/00927</td></tr>
</table>

| A. CLASSIFICATION OF SUBJECT MATTER |
|---|
| Int.Cl⁷ C07K 14/705, C12N 15/12, C12N 5/10, C12P 21/02, C07K 16/28, C12P 21/08, A61K 39/395, G01N 33/50, C12Q 1/00, C07K 14/46, C12Q 1/68 |

Int.Cl⁷ C07K 14/705, C12N 15/12, C12N 5/10, C12P 21/02, C07K 16/28, C12P 21/08, A61K 39/395, G01N 33/50, C12Q 1/00, C07K 14/46, C12Q 1/68

According to International Patent Classification (IPC) or to both national classification and IPC

**B. FIELDS SEARCHED**

Minimum documentation searched (classification system followed by classification symbols)

Int.Cl⁷ C07K 14/00~16/46, C12N 15/00~90, C12N 1/00~5/28, C12P 21/00~08, G01N 33/50~98, C12Q 1/00~70, A61K 31/00~48/00

Documentation searched other than minimum documentation to the extent that such documents are included in the fields searched

Electronic data base consulted during the international search (name of data base and, where practicable, search terms used)

SwissProt/PIR/GeneSeq, MEDLINE(STN),WPI(DIALOG),
Genbank/EMBL/DDBJ/GeneSeq, BIOSIS(DIALOG)

**C. DOCUMENTS CONSIDERED TO BE RELEVANT**

| Category* | Citation of document, with indication, where appropriate, of the relevant passages | Relevant to claim No. |
|---|---|---|
| PA | EP, 899332, A2 (SMITHKLINE BEECHAM CORP), 02 March, 1999 (02.03.99) & JP, 11056377, A & CA, 2224096, A | 1-22, 24-27 |
| A | EP, 867508, A2 (SMITHKLINE BEECHAM CORP), 03 September, 1998 (03.09.98) & US, 5874243, A & JP, 11028094, A | 1-22, 24-27 |
| EA | Gene, Vol.248, No 1-2, (May 2, 2000), p.183-189, Matumoto M. et al."The novel G-protein coupled receptor SALPR shares sequence similarity with somatostatin and angiotensin receptors." | 1-11,14-16, 19-22 |
| PA | Biochem.Biophys.Res.Commun., Vol.265, No.1, (Nov. 1999), p.123-129, Mori M. et al., "Urotensin II is the endogenous ligand of a G-protein-coupled orphan receptor,SENR (GPR14)." | 1-11,14-16, 19-22 |
| A | Brain Res.Mol.Brain Res., Vol.54, No.1, (Feb.1998), p.152-160, Donohue P.J. et al., "A human gene encodes a putative G protein-coupled receptor highly expressed in the central nervous system." | 1-11,14-16, 19-22 |

☐ Further documents are listed in the continuation of Box C.   ☐ See patent family annex.

| | | |
|---|---|---|
| * Special categories of cited documents: | "T" | later document published after the international filing date or priority date and not in conflict with the application but cited to understand the principle or theory underlying the invention |
| "A" document defining the general state of the art which is not considered to be of particular relevance | | |
| "E" earlier document but published on or after the international filing date | "X" | document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" document referring to an oral disclosure, use, exhibition or other means | | |
| "P" document published prior to the international filing date but later than the priority date claimed | "&" | document member of the same patent family |

| Date of the actual completion of the international search | Date of mailing of the international search report |
|---|---|
| 30 May, 2000 (30.05.00) | 20. 06. 2000 |

| Name and mailing address of the ISA/ | Authorized officer |
|---|---|
| Japanese Patent Office | |
| Facsimile No. | Telephone No. |

Form PCT/ISA/210 (second sheet) (July 1992)

## INTERNATIONAL SEARCH REPORT

| International application No. |
|---|
| PCT/JP00/00927 |

**Box I    Observations where certain claims were found unsearchable (Continuation of Item 1 of first sheet)**

This international search report has not been established in respect of certain claims under Article 17(2)(a) for the following reasons:

1. ☒ Claims Nos.: 23
   because they relate to subject matter not required to be searched by this Authority, namely:

   The subject matter of claim 23 corresponds to a diagnostic method
   for treatment of the human body.

2. ☐ Claims Nos.:
   because they relate to parts of the international application that do not comply with the prescribed requirements to such an
   extent that no meaningful international search can be carried out, specifically:

3. ☐ Claims Nos.:
   because they are dependent claims and are not drafted in accordance with the second and third sentences of Rule 6.4(a).

**Box II    Observations where unity of invention is lacking (Continuation of Item 2 of first sheet)**

This International Searching Authority found multiple inventions in this international application, as follows:

1. ☐ As all required additional search fees were timely paid by the applicant, this international search report covers all searchable
   claims.

2. ☐ As all searchable claims could be searched without effort justifying an additional fee, this Authority did not invite payment
   of any additional fee.

3. ☐ As only some of the required additional search fees were timely paid by the applicant, this international search report covers
   only those claims for which fees were paid, specifically claims Nos.:

4. ☐ No required additional search fees were timely paid by the applicant. Consequently, this international
   search report is restricted to the invention first mentioned in the claims; it is covered by claims Nos.:

**Remark on Protest**    ☐    The additional search fees were accompanied by the applicant's protest.

☐    No protest accompanied the payment of additional search fees.

Form PCT/ISA/210 (continuation of first sheet (1)) (July 1992)